# EUROPEAN PATENT APPLICATION

(11) **EP 3 811 945 A1**
(43) Date of publication of application: **28.04.2021**
(21) Application number: 19205268.6
(22) Date of filing: 25.10.2019
(51) Int. Cl.: A61K 31/505, A61K 31/506, A61K 31/5377, A61P 3/10, A61P 11/00, A61P 25/28, A61P 29/00, A61P 35/00, A61P 35/02, A61P 35/04, A61P 37/06, A61P 43/00

(54) **COMPOUNDS FOR TREATING AND PREVENTING GROWTH HORMONE RECEPTOR-DEPENDENT CONDITIONS**

(71) Applicant: UMC Utrecht Holding B.V., 3584 CM Utrecht (NL); Universiteit Utrecht Holding B.V., 3584 CM Utrecht (NL); Bimini Biotech BV, 1391 ED Abcoude (NL)
(72) Inventor: STROUS, Gerardus Jacobus Antonius Maria, 4175 AP HAAFTEN (NL); MOL, Jan Adrianus, 3994 ZP HOUTEN (NL); VAN DER VELDEN, Lieke Maria, 5271 ZL SINT-MICHIELSGESTEL (NL); KLUMPERMAN, Judith, 3628 BB KOCKENGEN (NL); MAAS, Petrus Emmanuel Marie, 3053 LB ROTTERDAM (NL); PIET, Dennis Patrick, 2904 BR CAPELLE AAN DEN IJSSEL (NL); DE KLERK-SPRENKELS, Nanda Elisabeth, 2642 DR PIJNACKER (NL); TIJHUIS, Johann Heinrich, 2613 AT DELFT (NL); VIËTOR, Hendrik Engelbertus, 1391 ED ABCOUDE (NL); MAURICE, Madelon Maria, 3731 HA DE BILT (NL); VAN DER KRIFT, Felix, 3531 CK UTRECHT (NL)
(74) Representative: Wensvoort, Gert

(57) **Abstract**

The invention relates to compounds, in particular pyrimidine-2,4-diamines, analogues and salts thereof and pharmaceutical compositions comprising pyrimidine-2,4-diamines analogues and salts thereof for use in the treatment and prevention of a disease, in particular a growth hormone receptor-dependent condition. The invention also relates to methods of using these compounds and compositions to treat physiological dsorders related to the amount or activity of growth hormone. In a particular embodiment, the invention relates to a compound according to formula 1 for use in the treatment or prevention of a disease in a subject

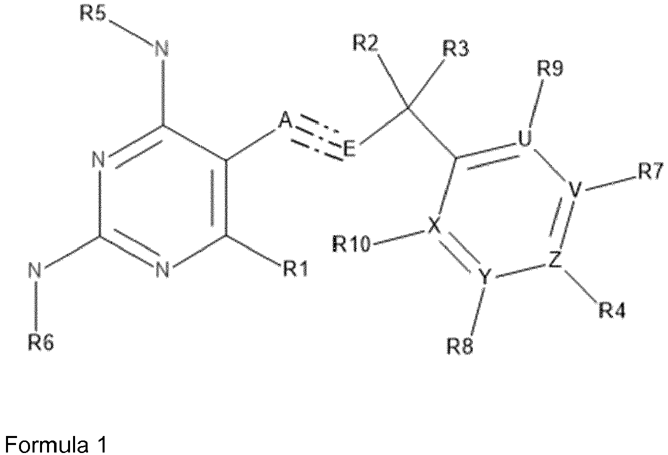

## Description

### Field of the invention

The present invention relates to compounds, in particular to pyrimidine-2,4-diamines, analogues and salts thereof and pharmaceutical compositions comprising pyrimidine-2,4-diamines analogues and salts thereof for use in the treatment and prevention of a disease, in particular a growth hormone receptor-dependent condition. The invention also relates to methods of using these compounds and compositions to treat physiological disorders related to the activity of growth hormone , more particularly for the treatment of cancer, preventing or treating cancer metastasis, treatment or prevention of diabetes, treatment of chronic inflammation such as rheumatoid arthritis and Crohn's disease, treatment of acromegaly, treatment or prevention of neurodegenerative diseases, treatment or prevention of idiopathic pulmonary disease and other occurrences of fibrosis, and the treatment or prevention of autoimmune diseases, such as Lupus erythematosus and macular degeneration, improving long- and short-term memory and increasing health span.

In particular, the invention relates to the treatment or prevention of cancer such as melanoma, acute myeloid leukemia, chronic lymphocytic leukemia, colorectal cancer, renal cell carcinoma, breast cancer, lung cancer, ovarian cancer, fallopian tube carcinoma, primary peritoneal carcinoma, cervical cancer, gastric cancer, liver cancer, pancreatic cancer, thyroid cancer, glioma, non-Hodgkin's lymphoma, and Hodgkin's lymphoma.

### Background of the invention

Growth hormone (GH) and insulin-like growth factor-1 (IGF-1) play a central role in development, differentiation, growth, and metabolism in mammals. The history of growth hormone (GH) has been described by Buchman, Bell and Kopchick (Buchman et al., 2018). The state-of-the-art of the GH field has been reviewed in Basu et al., 2018; Dehkhoda et al., 2018, and Ranke and Wit, 2018.

GH receptor (GHR) acts as a modulator of cellular metabolism, whose loss is not lethal, but results in sub-optimal health with short stature, decreased bone mineral density, decreased muscle strength, thin skin and hair, increased adiposity, and hepatic steatosis. Interestingly, people without GHR signaling live normal lives, but are highly resistant to cancer and diabetes type 2. In a well-studied Ecuadorian cohort of 100 persons, no cancer deaths were observed (Guevara-Aguirre et al., 2011). They also performed significantly better in memory tasks and had lower cognitive impairment compared to their unaffected relatives (Nashiro et al., 2017).

In adults, hypersecretion of GH causes acromegaly, and strategies that block the release of GH or that inhibit GHR activation are the primary forms of medical therapy for this disease. Overproduction of GH has also been linked to cancer and the microvascular complications that are associated with diabetes. However, studies to investigate the therapeutic potential of GHR antagonism in these diseases have been limited, most likely due to difficulty in accessing therapeutic tools to study the pharmacology of the receptor in vivo (Lu et al., 2019).

We set out to investigate if a small molecule can be defined that is capable to control the GH/IGF-1 axis.

### Summary of the invention

The present invention solves one or more problems of the prior art by providing in at least one embodiment, a compound or composition for treating diseases or conditions by causing inhibition of the synthesis of the growth hormone receptor (GHR). The compound according to this embodiment has formula 1 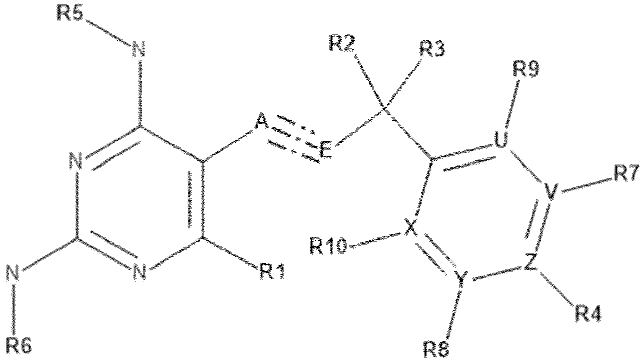 wherein
A and E represent carbon atoms with binding between A and E with a single bond or a double bond in E-configuration or a triple bond.
U, V, X, Y and Z represent carbon atoms or one of them represents an unsubstituted N to form a pyridine,
R¹ is (1C-3C)-alkyl or 3,3,3-trifluoropropyl,
R² is methyl and R³ is hydrogen or methyl, or R² and R³ form together with the carbon to which they are attached a cyclopropane, or R² and R³ form together with the carbon to which they are attached a cyclopentane,
R⁴, R⁷, R⁸, R⁹ and R¹⁰ are hydrogen, halogen, trifluoromethyl, methoxy, trifluoromethoxy, morpholine or methylsulfonyl,
R⁵ and R⁶ are hydrogen, ethyl or R⁵ or R⁶ are pyridin-2-ylmethyl,
or a pharmaceutically acceptable salt thereof.

### Legend to the figures

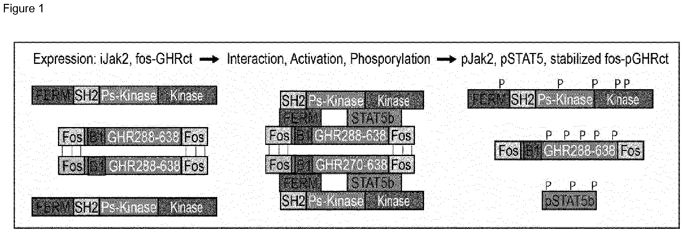

Figure 1. Diagram of the primary screening method for candidate compounds.
   Depicted are GHR fos-zipped cytosolic tails, amino acid 288-638 with fos zippers (fos), box-1 sequence (B1), and Jak2 (FERM, SH2, pseudokinase and kinase domains), as described in Nespital et al., 2016; Sedek et al, 2014). If the dimerized tails and Jak2 bind via FERM and Box-1, Jak2 gets activated and phosphorylates itself, the dimerized tails and STAT5b. After phosphorylation of specific tyrosine residues on GHR, Jak2 and STAT5, the complex dissociates; as fos-pGHRct is protease-resistant it accumulates in the cells. Unphosphorylated fos-GHRct is rapidly degraded. Steady state levels of Fos-pGHRct are lower if its synthesis, degradation or Jak2-interaction is changed by a candidate compound. The cell-based assay was performed in y2A cells carrying inducible Jak2 (iJak2) or binding-deficient iJak2 Y119E (as control).

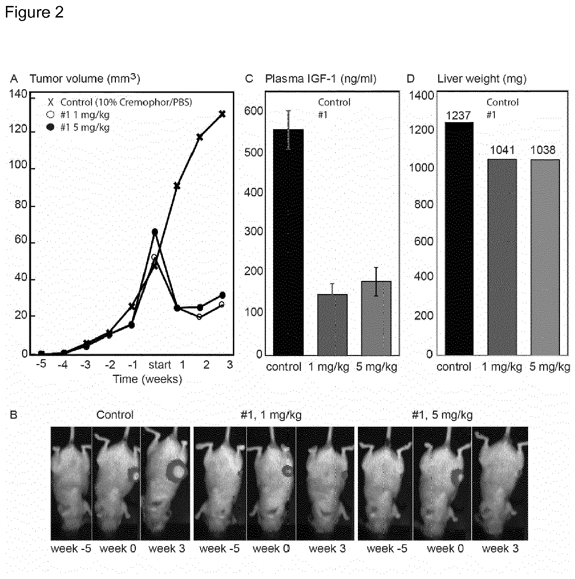

Figure 2. Effect of test compounds on mice, xenografted with MDA-MB-231 cells.
   Athymic nude BALB/c mice were transplanted with luciferase-expressing MDA-MB-231 cells at week -5. Starting at week 0, compound #1 was injected 3 times a week for 3 weeks; group size: 9, intraperitoneally (IP) at 1 or 5 mg/kg as indicated. Anesthetized mice were IP-injected with luciferin and the bioluminescence was imaged. The animals received the last drug injections 2 hours before they were euthanized, then blood was collected and livers were weighed. Mice treated with compound #1 showed a significantly lowered serum IGF-1 (27%) and liver weight (85%), whereas total body weights remained the same.
   Panel A: Quantification of bioluminescence imaging of mice treated with compound #1 as described in Vermeulen et al., 2013.
   Panel B: Tumor growth of control and mice treated with compound #1 using bioluminescence imaging (PhotonIMAGER, Biospace Lab, Paris, France).
   Panel C: Plasma IGF-1 levels of control and mice treated with compound #1.
   Panel D: Liver weights of control and mice treated with compound #1.
Figure 3: Effect of compound #1 on GHR/Jak/STAT functions in canine mammary organoids derived from tumor cells.
   Organoids were cultured in Matrigel in the presence of a variety of growth factors, Wnt activators and MPA. Organoids were treated for 48 h with 100 nM of compound #1 followed by staining with DAPI (nuclei) or specific antibodies against GHR or cytokeratin 8.
Figure 4A: Effect of compounds on GHR levels in Hek293T cells.
   GHR-expressing HEK293T_cells were transfected with Jak2 and treated overnight with Ruxolitinib (lane 1), DMSO (lane 2), 10 µM Compound #2 (lane 3), and 10 µM Compound #1 (lane 4) Ten minutes before lysis the cells were incubated with 90 ng GH/ml to activate the GHRs. The amounts of GHR both at the plasma membrane (PM) and in the endoplasmic reticulum (ER) were decreased in the cells treated with the compounds. In the presence of the Jak2 inhibitor, Ruxolitinib, the GHR protein levels were unchanged, but tyrosine phosphorylation (pY) of the GHR was inhibited (middle panel). Decrease of the pY signal in lanes 3 and 4 in the middle panel reflects the decrease of GHR protein levels (in the upper panel). The amounts of cells were identical across the experiment as shown by actin staining. *, irrelevant background staining.

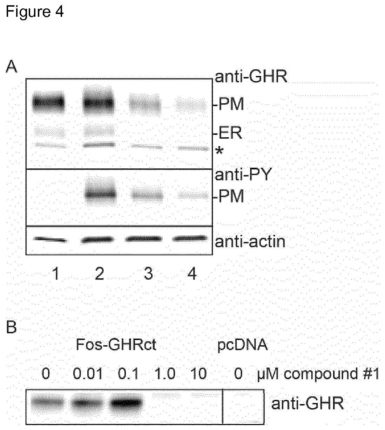

Figure 4B: Effect of compound #1 on the in vitro translation of fos-zippered GHR cytosolic tails in an HeLa cell cell-free system. pcDNA3 vector DNA expressing Fos-zippered GHR cytosolic tails as described in Nespital et al, 2016 was used to show that the compounds act on protein translation at the ribosomal level. Using increasing concentrations reveals that compound #1 is effective at concentrations > 0.1 µM. Translation of other proteins was not affected by the drugs (not shown). We used a cell-free translation system based on HeLa cell extract (Promega) Empty vector (pcDNA) was used as control. The blots were detected with an anti-GHR antibody raised in rabbits against the cytosolic tails.

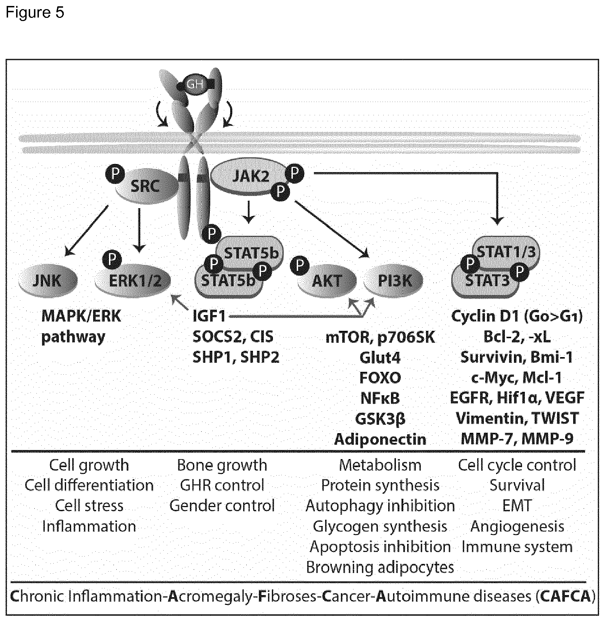

Figure 5: Schematic view of GHR signaling.
   Top panel, downstream signaling pathways with their main expressed genes and enzymatic activities; middle panel, resulting effect; lower panel, major diseased states if unbalanced.

### Detailed description of the invention

Reference is made to preferred compositions, embodiments, and methods of the present invention, which constitute the best modes of practicing the invention presently known to the inventors. The disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

As used herein, the singular form "a," "an," and "the" comprise plural referents unless the context clearly indicates otherwise. Reference to a component in the singular is intended to comprise a plurality of components. Where publications are referenced, the disclosures of these publications in their entireties are hereby incorporated by reference into this application to more fully describe the state of the art to which this invention pertains.

An extended number of studies in animals indicate the involvement of endocrine GH and IGF-1 in tumor growth promotion and demonstrate that effective therapeutic options for cancer treatment need to drastically lower serum IGF-1 (Chhabra, 2011). Attempts to successfully develop effective IGF-1 inhibitor failed, as IGF-1 (and somatostatin) act in an auto-inhibitory loupe with GH in the pituitary: interrupted IGF-1 signaling causes upregulation of the Jak/STAT pathway via high concentrations of GH in the blood. Early attempts to treat breast cancer include hypophysectomy (Jessiman et al., 1959) and application of somatostatin receptor ligands, such as octreotide, lanreotide, pasireotide, somatoprim, etc., that inhibit GH secretion (Theodoropoulou and Stalla, 2013). Treatment with the GH antagonist, Pegvisomant, is currently the only way to reduce GH activity (Basu et al., 2018).

Herein we describe the identification of compounds, in particular pyrimidine-2,4-diamines according to formula 1, as the first small molecular inhibitor of the integral GH/IGF-1 axis. 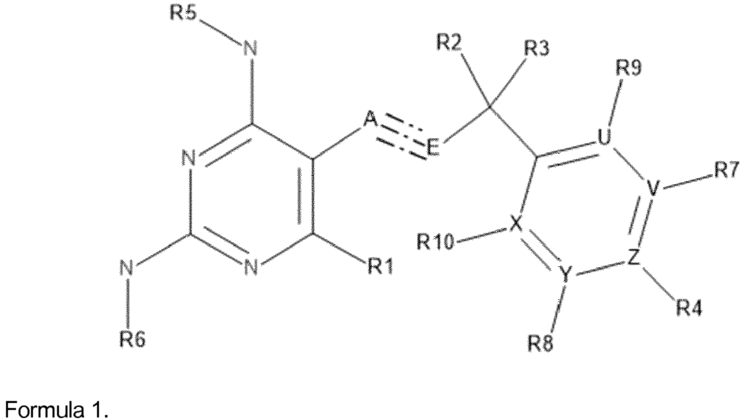 wherein
A and E represent carbon atoms with binding between A and E with a single bond or a double bond in E-configuration or a triple bond.
U, V, X, Y and Z represent carbon atoms or one of them represents an unsubstituted N to form a pyridine,
R¹ is (1C-3C)-alkyl or 3,3,3-trifluoropropyl,
R² is methyl and R³ is hydrogen or methyl, or R² and R³ form together with the carbon to which they are attached a cyclopropane, or R² and R³ form together with the carbon to which they are attached a cyclopentane,
R⁴, R⁷, R⁸, R⁹ and R¹⁰ are hydrogen, halogen, trifluoromethyl, methoxy, trifluoromethoxy, morpholine or methylsulfonyl,
R⁵ and R⁶ are hydrogen, ethyl or R⁵ or R⁶ are pyridin-2-ylmethyl,
or a pharmaceutically acceptable salt thereof.

These molecules act on the synthesis of the growth hormone receptor (GHR) and deplete cells from GH signaling activity. Consequently, mice treated with the molecule have low serum levels of IGF-1. In addition, the molecule inhibits the growth of human triple-negative breast cancer cells both in tissue culture and in xenografted mice.

We screened a 38,720-compound library and in two consecutive rounds of analogue selection we detected a small molecule that inhibited signaling via the GH receptor (GHR) and its downstream effectors Jak2 and STAT5. This molecule is shown as compound #1 in Table 1.
We then synthesized and tested several analogues of compound #1 according to formula 1 and came up with 28 more active compounds (table 1 and table 2). An active compound in this respect is herein defined as a compound according to formula 1 that shows either:
1. A lower plasma level of IGF-1 in a model of xenografted mice according to example 5,
2. Anti-tumor activity in a model of xenografted mice according to example 5,
3. Anti-proliferation activity with an EC50 value of 2000 nM or less (wherein less means a value smaller than 2000 nM) in an assay according to example 4,
4. A value of less than 1 in the viability assay according to example 7 on either MDA-MB-231 cells or MDA-MB-453 cells.

In this way a set of 28 compounds according to formula 1 were synthesized and tested (example 8). All 28 compounds showed the desired activity. These compounds prevent the translation of GHR (Figure 4) and the mice treated with these compounds showed the same phenotype as GHR knock-out mice (GHR-KO) and people with inherited Laron disease who lack a functional GHR (Laron, 2015). As a consequence of that, their length growth is hampered. Yet, they live normal lives, are fertile, and have a healthy normal life span. However, they do not suffer from cancer or diabetes type 2.

Numerous studies on GHR KO mice have confirmed these phenotypes. These mice are resistant to cancer incidence and progression. In addition, these mice have reduced serum IGF-1, increased insulin sensitivity, are resistant to High-Fat-Diet-induced diabetes, and to diabetic kidney disease, have a 21%-40% longer lifespan, a slower age-related neuromusculoskeletal deterioration, improved mitochondrial biogenesis, are resistant to oxidative stress, have less senescent cells, less fibrosis, less macrophage inflammation, lower hepatic inflammation and steatosis, increased brain size compared to body, increased brain NMDA-receptors, better long-and short-term memory, and better cognitive performance.

In humans, it is generally appreciated that the GH/IGF-1 axis is a driver for all these diseases and conditions. Consequently, a small molecule that is able to down-regulate the GH/IGF-1 axis is useful in the treatment or prevention of each of these conditions, as a principal drug or in combination with other treatments. The compounds and compositions disclosed herein may therefore be used to inhibit cancer and/or prevent/inhibit cancer metastasis, to prevent or cure diabetes, to act as geroprotectors and senolytics and to increase health span.

Harnessing the activity of GH has been a long-time goal. Initially, the actions were aimed at fighting the activity in GH-overproducing individuals called acromegaly. Recently, it became clear that the GH/IGF-1 axis constitutes a driver behind many conditions that shortens health-span in humans. Solid evidence shows that in many tissues cancer growth depends on the activity of the GH/IGF-1 axis.

Now it is generally accepted that both GH and IGF-1 are drivers of the major human cancers. In addition, GH/IGF-1 axis appears to have a positive role in onset and progress of other chronic diseases as indicated above,

There is only one GHR-based therapy using the GH-antagonist, namely pegvisomant. It is used in the treatment of acromegaly and in cancer cell lines. A major disadvantage of pegvisomant is that it only acts endocrine, while the cancer driver activity of GH occurs in autocrine mode.

Several efforts aim at the development of an IGF-1 inhibitor. The problem is that IGF-1 acts in an auto-inhibitory loop to control GH activity. If IGF-1 activity is artificially decreased, the levels of GH remain high, causing hyperstimulation of the Jak2/Stat and Src pathways, which in itself contribute to cancer and diabetes.

There are many efforts to inhibit the GH/IGF-1 axis downstream of their receptors, e.g. aimed at: Jak1, Jak2, STAT, AKT, mTOR, glucose control, or protein synthesis. They all have the disadvantage that they act on general 'house-hold' enzyme systems and lack specificity.

An exception is metformin. Metformin has been used as a safe and effective treatment for diabetes type 2 for over half a century, yet the precise mechanism of action of this drug remains elusive. The anti-hyperglycaemic properties of metformin are chiefly mediated by suppressing hepatic gluconeogenesis and it is generally accepted that this is achieved via inhibition of complex I in the mitochondrial respiratory chain. This impedes gluconeogenic flux, interferes with glucagon signaling and promotes activation of the major metabolic regulator AMPK. There are no other small molecules known to regulate the GH/IGF-1 axis

The drug development protocol described herein resulted in a number of pyrimidine-2,4-diamines that were found to be effective in inhibiting the translation of GHR. The assays as described in the examples were designed to find drugs that inhibit GHR signaling and was based on the hypothesis that inhibiting the GH/IGF-1 pathway will inhibit cancer growth.

The results of the mice experiments of Fig. 2A and 2B present the first evidence for this hypothesis. The drugs were also shown to decrease liver weight and IGF-1 levels, as in GHR knockout mice and Laron patients. GHR knock- out mice have low circulating IGF-1 and high GH levels due to the lack of GHR activity and less negative feedback of IGF-I on the pituitary gland (Zhou et al., 1997). Figures 2C and 2D show that the same phenotype was found in mice treated with the compounds and compositions according to the present invention. The animals of Fig. 2A received the last drug injections 2 h before they were euthanized, then blood was collected and livers were weighed. Only animalstreated with the compounds according to the invention showed significant effects. The drugs indeed lowered serum IGF-1 (27%) and liver weight (85%), while total body weights remained the same.

The results of Fig. 2 add another important piece of information: they prove that the compounds according to the invention work systemic on the GH/IGF-1 axis in mice, in addition to the human GHR system (as in the MDA-MB-231 cells), and the rabbit GHR (as in the primary screen and the transfected Hek293T cells).

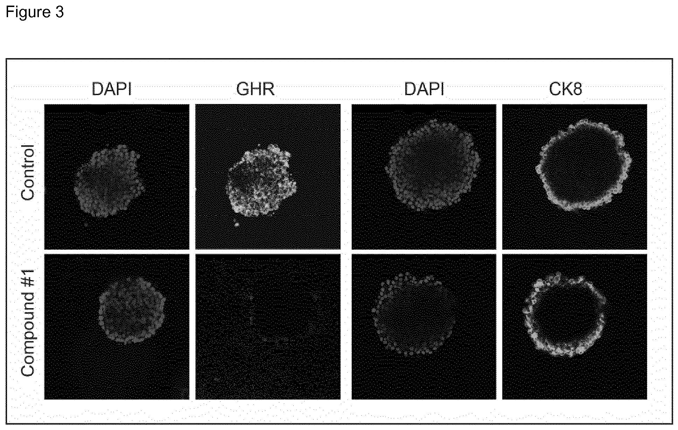

Next, we determined that also growth of dog breast cancer cells, grown as organoids, could be inhibited with the compounds according to the invention, in the best case (compound #1) with an EC50 of about 20 nM, see also figure 3.

Hence, the invention relates to the first medical use of a group of compounds according to formula 1 or a composition comprising such a compound in the treatment or prevention of a disease in a subject 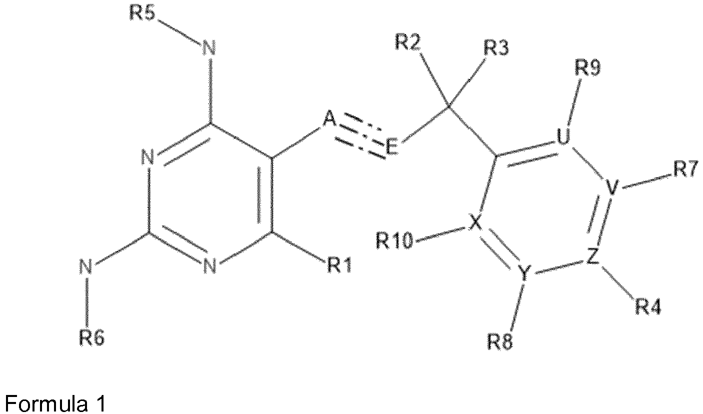 wherein
A and E represent carbon atoms with binding between A and E with a single bond or a double bond in E-configuration or a triple bond.
U, V, X, Y and Z represent carbon atoms or one of them represents an unsubstituted N to form a pyridine,
R¹ is (1C-3C)-alkyl or 3,3,3-trifluoropropyl,
R² is methyl and R³ is hydrogen or methyl, or R² and R³ form together with the carbon to which they are attached a cyclopropane, or R² and R³ form together with the carbon to which they are attached a cyclopentane,
R⁴, R⁷, R⁸, R⁹ and R¹⁰ are hydrogen, halogen, trifluoromethyl, methoxy, trifluoromethoxy, morpholine or methylsulfonyl,
R⁵ and R⁶ are hydrogen, ethyl or R⁵ or R⁶ are pyridin-2-ylmethyl,
or a pharmaceutically acceptable salt thereof.

One member of this group (compound #1) is known as a pesticide and described in WO 98/20878. To our knowledge, no medical treatment has been described for this group of compounds.

The compounds described herein are administered to the subject in a therapeutically effective amount so that growth hormone receptor dependent conditions may be treated or prevented. Examples of such conditions or diseases that may be treated or prevented are cancer, cancer metastasis, diabetes, chronic inflammation, rheumatoid arthritis, Crohn's disease, acromegaly, neurodegenerative diseases, idiopathic pulmonary disease, fibrosis, autoimmune diseases, Lupus erythematosus and macular degeneration. The compounds may also be used for improving long- and short-term memory and increasing health span.

The compounds as described herein may form pharmaceutically acceptable salts with both organic and inorganic acids or bases. For example, the acid addition salts of the basic compounds are prepared either by dissolving the free base in aqueous or aqueous alcohol solution or other suitable solvents containing the appropriate acid and isolating the salt by evaporating the solution.

Examples of pharmaceutically acceptable salts are hydrochlorides, hydrobromides, hydrosulfates, etc. as well as sodium, potassium, and magnesium, etc. salts. The invention includes the individual diastereomers or enantiomers, and the mixtures thereof. The individual diastereomers or enantiomers may be prepared or isolated by methods already well- known in the art.

Pharmaceutical compositions include the compounds as described herein or a salt thereof and a pharmaceutical carrier. Typically, the pharmaceutical compositions are divided into dosage units. Examples of dosage unit forms include, but are not limited to, pills, powders, tablets, capsules, aqueous and non-aqueous oral solutions and suspensions, and parenteral solutions.

Examples of suitable pharmaceutical carriers include, but are not limited to, water, sugars (e.g., lactose and sucrose), starches (e.g., corn starch and potato starch), cellulose derivatives (e.g., sodium carboxymethyl cellulose, and methyl cellulose), gelatin, talc, stearic acid, magnesium stearate, vegetable oils (e.g., peanut oil, cottonseed oil, sesame oil, olive oil, etc.), propylene glycol, glycerin, sorbitol, polyethylene glycol, water, agar, alginic acid, saline, and other pharmaceutically acceptable materials.

The percentage of the active ingredients in the pharmaceutical compositions can be varied within wide limit. For example, a concentration of at least 10% in a solid composition and at least 2% in a primary liquid composition is used.

Routes of administration of the compound or its salts are oral or parenteral. For example, a useful intravenous dose is between 1 and 50 mg and a useful oral dosage is between 5 and 800 mg.

Compounds as described herein may advantageously be used as a medicament for treating a disease, in particular when the disease is a growth hormone receptor-dependent disease.

The term "growth hormone receptor dependent disease" is used herein to indicate a disease that is associated with an increased amount or activity of growth hormone receptor or caused by an increased amount or activity of growth hormone receptor.

Moreover, the term "growth hormone receptor dependent disease" may also indicate a disease that is associated with an altered activity of the growth hormone receptor or caused by a locally increased amount or activity of growth hormone receptor.

More in particular, the term "growth hormone receptor dependent disease" is used herein to indicate a disease selected from the group consisting of cancer, cancer metastasis, diabetes, chronic inflammation, rheumatoid arthritis, Crohn's disease, acromegaly, neurodegenerative diseases, idiopathic pulmonary disease, fibrosis, autoimmune diseases, Lupus erythematosus and macular degeneration. The compounds may also be used for improving long- and short-term memory and increasing health span.

The compounds as disclosed and described herein may be used for treatment and/or prevention of diseases as disclosed herein wherein treatment or prevention is selected from the group consisting of the treatment or prevention of cancer, preventing cancer metastasis, treatment or prevention of diabetes, treatment of chronic inflammation such as rheumatoid arthritis and Crohn's disease, treatment of acromegaly, treatment or prevention of neurodegenerative diseases, treatment and prevention of idiopathic pulmonary disease and other occurrences of fibrosis, treatment and prevention of autoimmune diseases, such as Lupus erythematosus and macular degeneration, improving long- and short-term memory and increasing health span.

Preferred use is the use of a compound as disclosed herein for the treatment or prevention of cancer. Preferably, it is used to prevent metastasis of cancer cells.

Particularly preferred is the use of a compound as described herein in the treatment of cancer wherein the cancer is selected from the group consisting of melanoma, acute myeloid leukemia, chronic lymphocytic leukemia, colorectal cancer, renal cell carcinoma, breast cancer, lung cancer, ovarian cancer, fallopian tube carcinoma, primary peritoneal carcinoma, cervical cancer, gastric cancer, liver cancer, pancreatic cancer, thyroid cancer, glioma, non-Hodgkin's lymphoma, and Hodgkin's lymphoma.

In a preferred embodiment, the invention relates to a compound as disclosed above, wherein R5 is pyridin-2-ylmethyl and its medical use as disclosed herein.

In a further preferred embodiment, the invention relates to a compound as disclosed above wherein R5, R6, R7, R8 and R9 are hydrogen and its medical use as disclosed herein.

In an even further preferred embodiment, the invention relates to a compound as described above wherein R1 is methyl, R2 and R3 form together with the carbon to which they are attached a cyclopropane and R4 is chloro or trifluoromethyl.

In an even further preferred embodiment, the invention relates to a compound selected from the group consisting of 6-methyl-5-[2-{1-[4-(trifluoromethyl)phenyl]cyclopropyl}ethenyl]pyrimidine-2,4-diamine, 5-{[1-(4-chlorophenyl)cyclopropyl]ethynyl}-6-methylpyrimidine-2,4-diamine, 5-{[1-(4-chlorophenyl)cyclopropyl]ethynyl}-6-methylpyrimidine-2,4-diamine, 5-[3-(4-fluorophenyl)butyl]-6-methylpyrimidine-2,4-diamine, 5-{3-methyl-3-[4-(trifluoromethyl)phenyl]but-1-en-1-yl}-6-(3,3,3-trifluoropropyl)pyrimidine-2,4-diamine, 6-methyl-5-[3-methyl-3-[4-(trifluoromethyl)phenyl]but-1-en-1-yl]-N4-[(pyridin-2-yl)methyl]pyrimidine-2,4-diamine, 6-methyl-5-f 3-methyl-3-[4-(trifluoromethyl)phenyl]but-1-en-1-yl}-N2-[(pyridin-2-yl)methyl]pyrimidine-2,4-diamine and 6-methyl-5-{3-[4-(trifluoromethyl)phenyl]but-1-en-1-yl}pyrimidine-2,4-diamine and the medical uses thereof as described herein..

The invention also relates to compositions comprising a compound as described herein and their medical uses.

The term "subject" as used herein is intended to refer to a human or an animal, a mammal, a primate, a higher primate, a sheep, a dog, a rodent, a mouse, a rat, a guinea pig, a goat, a pig, a cat, a rabbit, or a cow.

**Table 1**

| **No: (#)** | **Name** | **Structure** | **EC50 (nM)** |
|---|---|---|---|
| 1 | 6-methyl-5-[2-{1-[4-(trifluoromethyl)phenyl]cyclopropyl}ethenyl]pyrimidine-2,4-diamine | 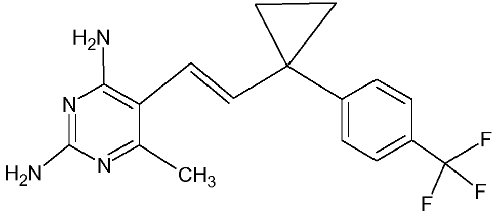 | 30 |
| 2 | 5-{[1-(4-chlorophenyl)cyclopropyl]ethynyl}-6-methylpyrimidine-2,4-diamine | 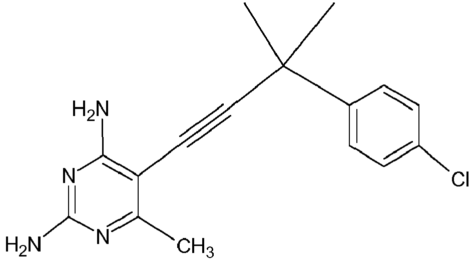 | 80 |
| 3 | 5-[3-(4-fluorophenyl)butyl]-6-methylpyrimidine-2,4-diamine | 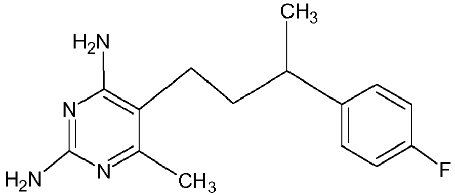 | 200 |
| 4 | 5-{3-methyl-3-[4-(trifluoromethyl)phenyl]but-1-en-1-yl}-6-(3,3,3-trifluoropropyl)pyrimidine-2,4-diamine | 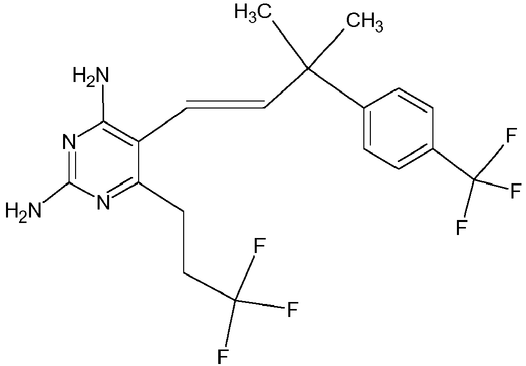 | 400 |
| 5 | 6-methyl-5-[3-methyl-3-[4-(trifluoromethyl)phenyl]but-1-en-1-yl]-*N*⁴-[(pyridin-2-yl)methyl]pyrimidine-2,4-diamine | 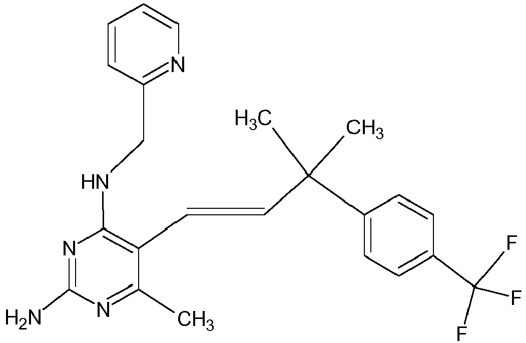 | 800 |
| 6 | 6-methyl-5-{3-methyl-3-[4-(trifluoromethyl)phenyl]but-1-en-1-yl}-*N*²-[(pyridin-2-yl)methyl]pyrimidine-2,4-diamine | 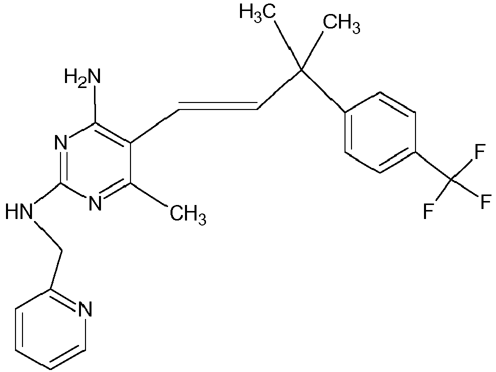 | 2000 |

The EC50 refers to the inhibitory effect of the compounds on the proliferation of triple negative human breast cancer cells (MDA-MB-231), tested after 24, 48, 72h in cell culture.

| **Table 2** | | | | |
|---|---|---|---|---|
| **No : (#)** | **Structure** | **Relative viability MDA-MB-231** | **relative viability MDA-MB-453** | **name** |
| 3 | 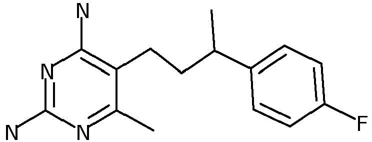 | 0.877 | 0.827 | 5-[3-(4-fluorophenyl)butyl]-6-methyl-2,4-pyrimidinediamine |
| 4 | 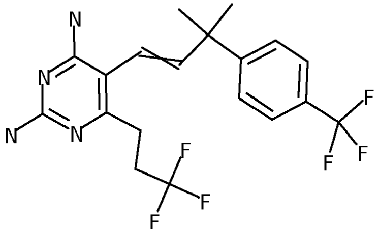 | 1.151 | 0.789 | 2-amino-5-{3-methyl-3-[4-(trifluoromethyl)phenyl]-1-butenyl}-6-(3,3,3-trifluoropropyl)-4-pyrimidinylamine |
| 5 | 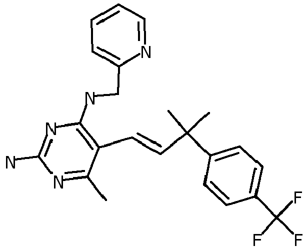 | 0.741 | 0.207 | N-(2-amino-6-methyl-5-{3-methyl-3-[4-(trifluoromethyl)phenyl]-1-butenyl}-4-pyrimidinyl)-N-(2-pyridinylmethyl)amine |
| 7 | 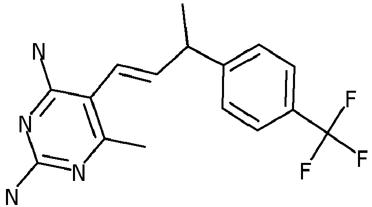 | 0.942 | 0.540 | 2-amino-6-methyl-5-{3-[4-(trifluoromethyl)phenyl]-1-butenyl}-4-pyrimidinylamine |
| 8 | 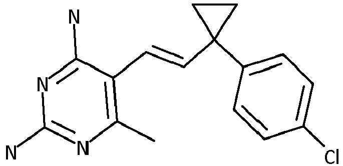 | 0.912 | 0.495 | 5-[2-[1-(4-chlorophenyl)cyclopropyl]vinyl]-6-methylpyrimidine-2,4-diamine |
| 9 | 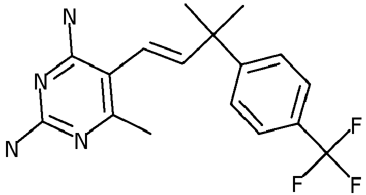 | 0.918 | 0.389 | 2-amino-6-methyl-5-{3-methyl-3-[4-(trifluoromethyl)phenyl]-1-butenyl}-4-pyrimidinylamine |
| 11 | 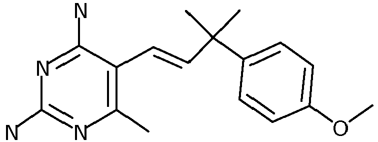 | 0.875 | 0.487 | 2-amino-5-[3-(4-methoxyphenyl)-3-methyl-1-butenyl]-6-methyl-4-pyrimidinylamine |
| 12 | 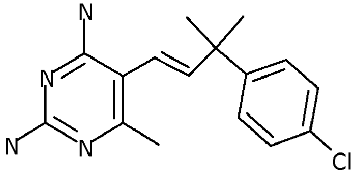 | 0.933 | 0.565 | 2-amino-5-[3-(4-chlorophenyl)-3-methyl-1-butenyl]-6-methyl-4-pyrimidinylamine |
| 13 | 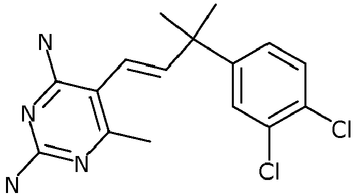 | 0.796 | 0.489 | 2-amino-5-[3-(3,4-dichlorophenyl)-3-methyl-1-butenyl]-6-methyl-4-pyrimidinylamine |
| 14 | 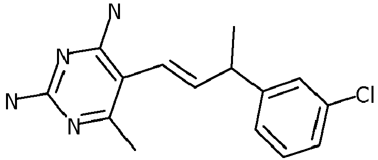 | 0.905 | 0.619 | 2-amino-5-[3-(3-chlorophenyl)-1-butenyl]-6-methyl-4-pyrimidinylamine |
| 15 | 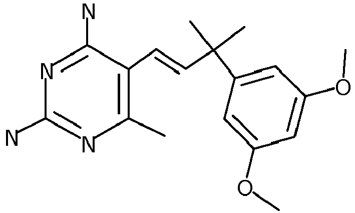 | 0.918 | 0.667 | 5-[3-(3,5-dimethoxyphenyl)-3-methyl-1-butenyl]-6-methyl-2,4-pyrimidinediamine |
| 16 | 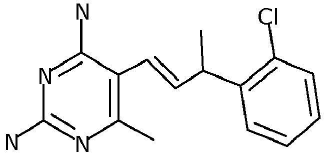 | 0.737 | 0.617 | 2-amino-5-[3-(2-chlorophenyl)-1-butenyl]-6-methyl-4-pyrimidinylamine |
| 17 | 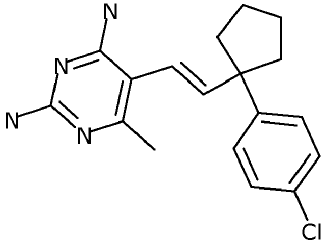 | 0.749 | 0.640 | 2-amino-5-{2-[1-(4-chlorophenyl)cyclopentyl]vinyl}-6-methyl-4-pyrimidinylamine |
| 18 | 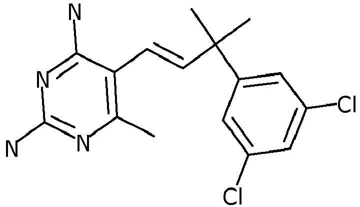 | 0.897 | 0.789 | 2-amino-5-[3-(3,5-dichlorophenyl)-3-methyl-1-butenyl]-6-methyl-4-pyrimidinylamine |
| 19 | 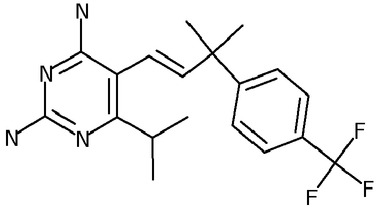 | 0.898 | 0.806 | 6-isopropyl-5-{3-methyl-3-[4-(trifluoromethyl)phenyl]-1-butenyl}-2,4-pyrimidinediamine |
| 20 | 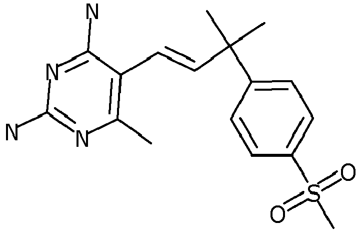 | 0.642 | 0.637 | 2-amino-6-methyl-5-{3-methyl-3-[4-(methylsulfonyl)phenyl]-1-butenyl}-4-pyrimidinylamine |
| 21 | 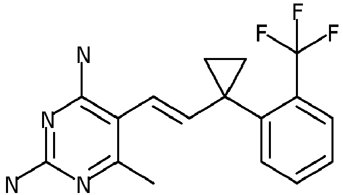 | 0.879 | 0.529 | 6-methyl-5-[2-[1-[2-(trifluoromethyl)phenyl]cyclopropyl]vinyl]pyrimidine-2,4-diamine |
| 22 | 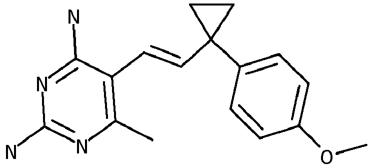 | 0.853 | 0.421 | 5-[2-[1-(4-methoxyphenyl)cyclopropyl]vinyl]-6-methylpyrimidine-2,4-diamine |
| 23 | 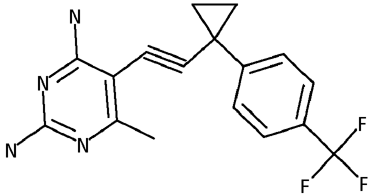 | 1.073 | 0.717 | 6-methyl-5-[2-[1-[4-(trifluoromethyl)phenyl]cyclopropyl]ethynyl]pyrimidine-2,4-diamine |
| 24 | 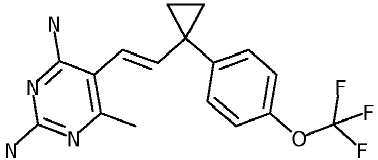 | 0.878 | 0.392 | 6-methyl-5-[2-[1-[4-(trifluoromethoxy)phenyl]cyclopropyl]vinyl]pyrimidine-2,4-diamine |
| 25 | 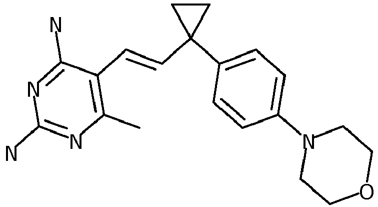 | 0.916 | 0.405 | 6-methyl-5-[2-[1-(4-morpholinophenyl)cyclopropyl]vinyl]pyrimidine-2,4-diamine |
| 26 | 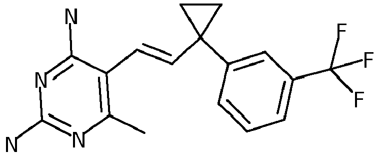 | 1.028 | 0.632 | 6-methyl-5-[2-[1-[3-(trifluoromethyl)phenyl]cyclopropyl]vinyl]pyrimidine-2,4-diamine |
| 27 | 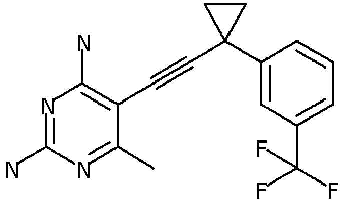 | 1.051 | 0.730 | 6-methyl-5-[2-[1-[3-(trifluoromethyl)phenyl]cyclopropyl]ethynyl]pyrimidine-2,4-diamine |
| 28 | 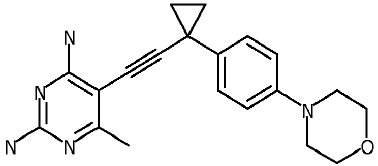 | 1.073 | 0.701 | 6-methyl-5-[2-[1-(4-morpholinophenyl)cyclopropyl]ethynyl]pyrimidine-2,4-diamine |
| 29 | 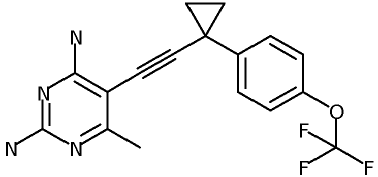 | 0.955 | 0.626 | 6-methyl-5-[2-[1-[4-(trifluoromethoxy)phenyl]cyclopropyl]ethynyl]pyrimidine-2,4-diamine |

### Examples

### Example 1: In vitro screening method

In our previous studies (Nespital et al., 2016; Sedek et al., 2014) we showed that soluble fos- zipped GHR cytosolic tails (fos-GHRct) can serve as signaling complexes when co-expressed with Jak2 (Figure 1). In this system, Fos-GHRct expressed without Jak2 is immediately degraded by the ubiquitin system. Phosphorylated fos-GHRct (fos-pGHRct) detaches from Jak2 and unzips. Only unzipped fos-pGHR cytosolic tail are protected from proteasomal degradation and accumulate in the cell. This system therefore offers a very sensitive cell-based assay for high-throughput screening with 4 functional parameters, namely: fos-pGHRct using both anti-GHR (a) and anti-pY (b), as well as anti-pJak2 (c) and anti-pSTAT5b (d) (Figure 1).

The assay can be used to screen for 6 different activities that will lead to a decreased or abolished signal: if a candidate compound (1) inhibits Jak2 kinase activity, (2) inhibits the fos-GHRct synthesis, (3) inhibits the fos-GHRct:Jak2 interaction, (4) induces fos-pGHRct degradation, (5) induces Jak2 degradation, and (6) induces fos-pGHRct dephosphorylation. Each of these 6 possibilities will result in lower signals a-d mentioned above (Nespital et al., 2016; Sedek et al., 2014). In the primary screen we used y2A cells with inducible Jak2 (iJak2) or iJak2-119E as binding-deficient Jak2 control (Figure 1).

### Example 2: Flow cytometer-based assay

109 primary hits from the assay described in example 1 were selected and the best 17 were tested in a flow cytometer-based assay of whole human blood, supplemented with human IM-9 lymphoblasts that have detectable levels of endogenous GH receptor (Dr. van Agthoven, Beckman-Coulter, Marseille, paid service) (Malergue et al., 2015).

This allowed simultaneous testing of GH- and GM-CSF activity. The GH/GM-CSF cytometric test added two important criteria to GH signaling: The test prioritized compounds that target the GH-driven signaling at the expense of another Jak2-dependent cytokine signaling (GM-CSF); equally important: it selected compounds that exert a rapid effect on the GH/IGF-axis as the response time for STAT5 phosphorylation was limited to 20-30 min. Given the short resident time of the GHR both in the synthesis (endoplasmic reticulum) and the signaling compartment (the plasma membrane (PM) of less than 30 min, candidate hits must have an immediate and strong effect on the signaling capacity via the PM. This screening yielded 10 compounds out of the original 109 primary hits.

### Example 3: Screening with Hek293T cells expressing GHR

Analogues of these 10 compounds were synthesized and 122 analogues were tested in Hek293T cells expressing GHR. GH-induced GH-GHR-Jak2 signaling was probed by GHR pY staining in concentration series (0-20 µM, 1 h drug-treated, 10 min GH). Based on data combined with again the GH/GM-CSF cytometric test, we selected 2 hits (Compounds #1 and #2) that both inhibited the phosphorylation of fos-GHRct (fos-pGHRct), of the active site of Jak2 (p1007/1008) and of STAT5 (pSTAT5) and were inactive towards GM-CSF signaling. Compound #1 showed the same potential (EC50 ∼ 0,3 µM) as Ruxolitinib (EC50, 0.1-0.5 µM) (Furqan et al., 2013). Ruxolitinib is a Jak1/Jak2 inhibitor that is being used for the treatment of intermediate or high-risk myelofibrosis, a type of myeloproliferative disorder that affects the bone marrow, and for polycythemia vera when there has been an inadequate response to or intolerance of hydroxyurea.

A set of 28 compounds according to formula 1 was then synthesized and tested for activity.

### Example 4: Proliferation test

We used the triple-negative human breast cancer line, MDA-MB-231 and quantified DNA in vital cells. Dose-response curves were made after 24, 48 & 72 h. The 28 compounds inhibited the cell line, with EC50 values varying from 30-2000 nM (Table 1). The compound with the lowest EC50 (Compound #1) was tested *in vivo.*

### Example 5: In vivo test system in mice

Mice were xenografted with MDA-MB-231 cells as described. This cell line was chosen as it is GH-responsive, pegvisomant can block doxorubicin-induced apoptosis, it lacks E-cadherin due to hypermethylation, proliferates fast, has a basal-like expression profile and originates from infiltrating ductal breast cancer (Christgen and Derksen, 2015; Hollestelle et al., 2013; Minoia et al., 2012).

Patients with this type of breast cancer, not driven by epidermal growth factor receptor 2 (HER-2), estrogen receptors (ER), or progesterone receptors (PR) have a poor prognosis.

We used a protocol of 3 injections per week (1 and 5 mg/kg) during 3 weeks (9 mice/group). The treatment started after 5 weeks at a tumor volume of about 60 mm³, and the animals were killed after 3 weeks at a tumor volume of 130 mm³ in the control group. The drug had an immediate effect on the tumors: growth halted and the tumor sizes were reduced (Figure 2A and 2B).

We also showed that the mice treated with compound #1 had a decreased level of plasma IGF-1 (figure 2C) and a reduced liver weight (figure 2D).

Neither control nor xenografted animals showed any discomfort during the 3-week treatments.

### Example 6: In vitro test system in dogs

Canine mammary organoids were derived from tumor cells and cultured in Matrigel in the presence of a variety of growth factors, Wnt activators and MPA (Timmermans-Sprang et al., 2017). Organoids were treated for 48 h with 100 nM of compound #1, followed by staining with DAPI (nuclei) or specific antibodies against GHR or cytokeratin 8 (CK8). The results are shown in figure 3.

### Example 7: Viability assay on MDA-MB-231 and MDA-MB-453 cells

Compounds were assessed for effects on viability of the breast cancer cell lines MDA-MB-231 and MDA-MB-453. The cells were incubated with the compounds for 46 hours at a concentration of 5 uM. DMSO was used as vehicle control. Cell viability was assessed after approximately 46 hours by using a luminescent cell viability assay. This assay generates a luminescent signal, proportional to the amount of ATP that is present as a readout of metabolically active cells. Relative viability was calculated by expressing the luciferase signal as a value normalized to untreated cells, meaning that the measured luciferase signal in untreated cells equals 1.

### Example 8: Synthesis of small molecules.

General scheme for the synthesis of vinylpyrimidine-2-4-diamines:

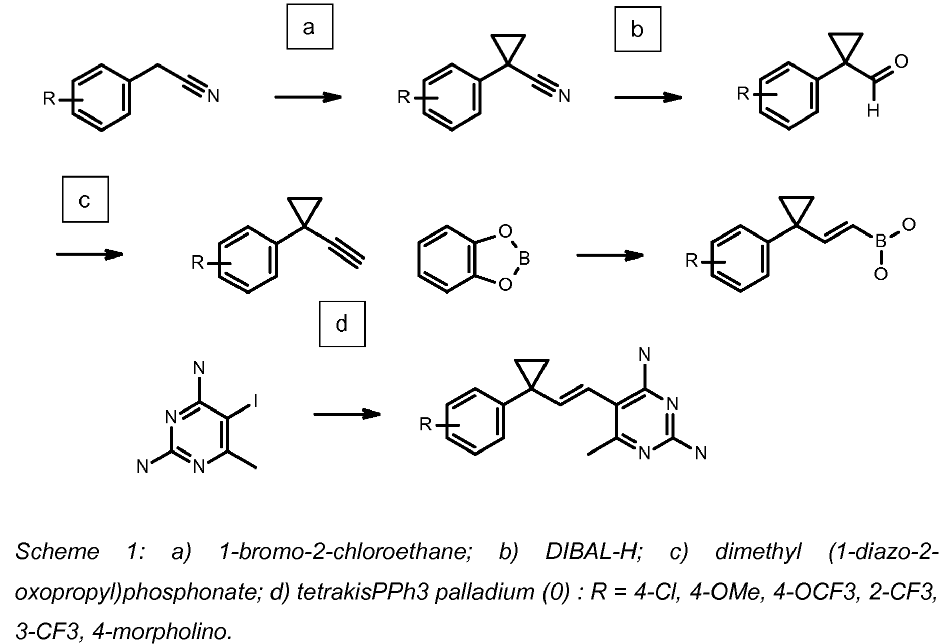

### Synthesis of compound #1.

The synthesis of compound #1 was performed as described in WO 98/20878.

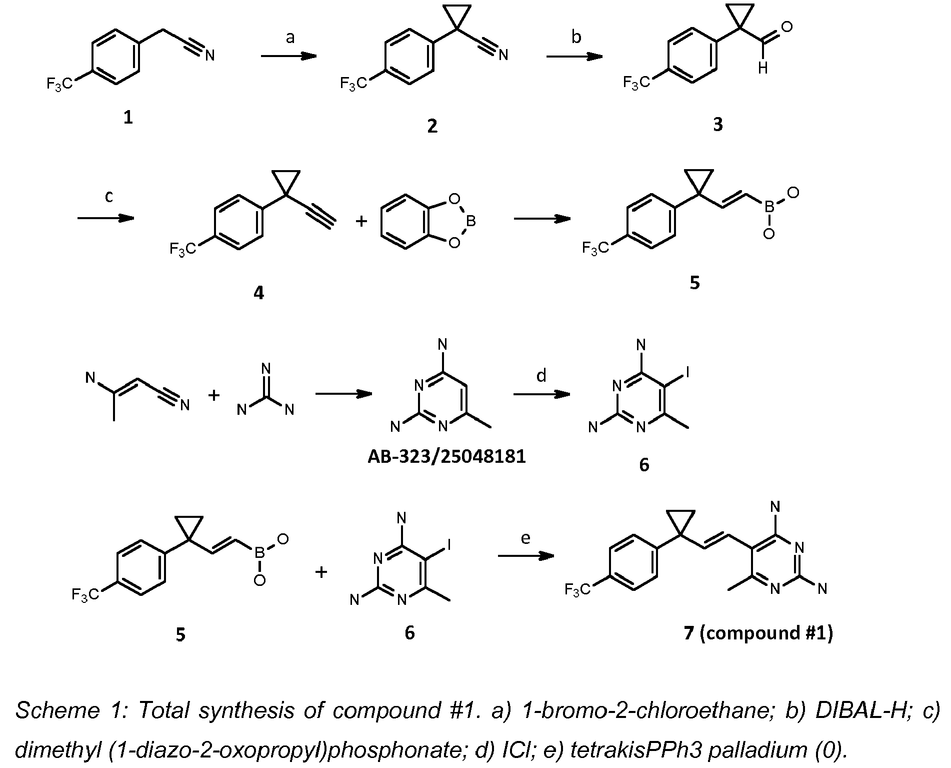

### Experimental part:

***1-[4-(trifluoromethyl)phenyl]cyclopropane carbonitrile (2):*** A mixture of benzyl triethylammonium chloride (137mg, 0.58mmol), 1-bromo-2-chloroethane (3.65mL, 43.75mmol), **1** (5.40g, 29.17mmol) and NaOH (7.00g, 175mmol) in 7ml of water was stirred at 50°C under a nitrogen atmosphere. Additional water was added to facilitate stirring if needed. After 16h TLC (10% EtOAc in PE (40-60)) indicated complete consumption of the starting material. The reaction mixture was diluted with EtOAc and washed twice with water. The combined aqueous layers were extracted with EtOAc and the combined organic layers were washed with brine, dried over MgSO₄ and evaporated to give **2** (6.21 g, 29.17mmol, 100%) as a red liquid which crystalizes on standing. ¹H NMR (400 MHz, *CDCl₃)* δ ppm 7.62 (d, 2H), 7.40 (d, 2H), 1.69-1.97 (m, 2H), 1.33-1.53 (m, 2H).

***1-[4-(trifluoromethyl)phenyl]cyclopropane carbaldehyde (3):*** To a solution of **2** (4.99g, 23.6mmol) in 20mL of DCM, cooled on an ice bath under a nitrogen atmosphere, DIBAL-H (1M in DCM) (5.04g, 35.5mL, 35.5mmol) was added dropwise. After the reaction mixture was stirred for 16h, TLC (10% EtOAc in PE (40-60)) indicated complete consumption of the starting material. 40mL 1N HCI solution was added dropwise to the reaction mixture at 0°C. Additional DCM was added to facilitate stirring. The layers were separated, and the organic fraction was washed twice with water. The combined aqueous layers were extracted with DCM and the combined organic layers were washed with brine, dried over MgSO₄ and evaporated. The residue was purified by flash column chromatography (silica, 50%DCM/PE (40/60)) to give **3** (3.72g, 17.37mmol, 74%) as a yellow liquid which crystallizes on standing. ¹H NMR (400 MHz, *CDCl₃*) δ ppm 9.16 (s, 1H), 7.53-7.68 (m, 2H), 7.30-7.50 (m, 2H), 1.54-1.69 (m, 2H), 1.33-1.52 (m, 2H).

***1-(1-ethynylcyclopropyl)-4-(trifluoromethyl)benzene (4)***: To a solution of **3** (6.03g, 28.2mmol) in 5mL MeOH, cooled on an ice bath under a nitrogen atmosphere, a solution of dimethyl (1-diazo-2-oxopropyl)phosphonate (5.5mL, 36.6mmol) in 5mL MeOH was added followed by the addition of K₂CO₃ (8.17g, 58.8mmol). After stirring 16h TLC (5% EtOAc in PE (40-60)) indicated complete consumption of the starting material. The reaction mixture was filtered over celite and the cake was washed with Et₂O. The filtrate was washed twice with water. The combined aqueous layers were extracted with Et₂O and the combined organic layers were washed with brine, dried over MgSO₄ and evaporated. The residue was purified by flash column chromatography (silica, PE (40-60)) to give **4** (5.34g, 25.42mmol, 90%) as a colourless oil. ¹H NMR (400 MHz, *CDCl₃)* δ ppm 7.55 (m, 2H), 7.43 (m, 2H), 2.16 (s, 1H), 1.40-1.64 (m, 2H), 1.16-1.40 (m, 2H).

***[2-[1-[4-(trifluoromethyl)phenyl]cyclopropyl]vinyl]boronic acid (5)**:* To a solution of **4** (1.55g, 7.38mmol) in 10mL THF a solution of catecholborane (0.97g, 0.87mL, 8.12 mmol) in 5mL THF was added and the resulting mixture was stirred for 6h at 70°C. TLC (30% EtOAc in PE (40-60)) indicated complete consumption of the starting material after which 50mL of water was added to the reaction mixture followed by stirring for 16h at room temperature. The reaction mixture was diluted with EtOAc and washed twice with water. The combined aqueous layers were extracted with EtOAc and the combined organic layers were washed with brine, dried over MgSO₄, evaporated to give a mixture of catechol and **5** (2.32g) as a brown solid. ¹H NMR (400 MHz, *CDCl₃*) δ ppm 7.50-7.62 (m, 2H), 7.31-7.49 (m, 2H), 6.58 (d, 1H), 4.93 (d, 1H), 1.12-1.33 (m, 4H).
***5-iodo-6-methyl-pyrimidine-2,4-diamine (6)*:** To a solution of AB-323/25048181 (1.50g, 12.06mmol) in 15mL AcOH a solution of ICI (2.54g, 15.64mmol) in 15mL AcOH was added. The reaction mixture was stirred at room temperature for 3h after which TLC (EtOAc) indicated complete consumption of the starting material. The reaction was basified with a 4N NaOH solution and extracted twice with EtOAc. The combined organic layers are washed with brine, dried over MgSO₄ and evaporated to give **6** (2.70g, 10.80mmol, 89%) as a brown solid. ¹H NMR (400 MHz, *CDCl₃*) δ ppm 5.14 (br. s., 2H), 4.70 (br. s., 2H), 2.44 (s, 3H).

***6-methyl-5-[2-[1-[4-(trifluoromethyl)phenyl]cyclopropyl]vinyl]pyrimidine-2,4-diamine (7, compound #1):* 6** (264mg, 1.05 mmol) was added to a solution of **5** (456mg) in 15mL of THF and the reaction mixture is purged with nitrogen. 5mL of a nitrogen purged stock solution of 2N Na₂CO₃ solution was added to the reaction mixture. The air was evacuated under high vacuum and filled with a nitrogen atmosphere. This procedure was repeated twice after which tetrakisPPh3 palladium (0) (277mg, 0.24mmol) was added. The reaction was stirred at 70°C for 16h under a nitrogen atmosphere. TLC (20% i-PrOH/EtOAc) indicated complete consumption of starting material. The reaction mixture was diluted with EtOAc and washed twice with water. The combined aqueous layers were extracted with EtOAc and the combined organic layers were washed with brine, dried over MgSO₄ and evaporated. The residue was purified by column chromatography (silica, 20% i-PrOH/EtOAc) to give a mixed fraction of **7** (compound #1) and **6,** as a yellow solid. The mixture is dissolved in 5mL isopropanol and injected into 150mL demineralized water. The formed solids are filtered over a glass filter and dried to give pure **7** (compound #1, 184mg, 0.55mmol, 46%). ¹H NMR (400 MHz, *CDCl₃*) δ ppm 7.61 (m, 2H), 7.47 (m, 2H), 5.71 (s, 1H), 5.66 (s, 1H), 4.64 (br. s., 2H), 4.57 (br. s., 2H), 2.13 (s, 3H), 1.17-1.32 (m, 2H), 1.05-1.17 (m, 2H).

### Synthesis of 5-[2-[1-(4-chlorophenyl)cyclopropyl]vinyl]-6-methylpyrimidine-2,4-diamine (GHR_0011) as a general example

### 1-(4-chlorophenyl)cyclopropanecarbonitrile:

A mixture of benzyl triethylammoniumchloride (133mg, 0.58mmol), 1-bromo-2-chloroethane (6.29g, 43.83mmol), 2-(4-chlorophenyl)acetonitrile (4.43g, 29.22mmol) and NaOH (7.00g, 175mmol) in 7ml of water was stirred at 50°C under a nitrogen atmosphere. Additional water was added to facilitate stirring if needed. After 16h TLC (15% EtOAc in PE (40-60)) indicated complete consumption of the starting material. The reaction mixture was diluted with EtOAc and washed twice with water. The combined aqueous layers were extracted with EtOAc and the combined organic layers were washed with brine, dried over MgSO₄ and evaporated to give 1-(4-chlorophenyl)cyclopropanecarbonitrile (4.85g, 29.17mmol, 100%) as a red liquid which crystalizes on standing. ¹H NMR (400 MHz, CDCl₃) δ ppm 7.32 (d, 2H), 7.22 (d, 2H), 1.74 (m, 2H), 1.38 (m, 2H).

### 1-[4-(trifluoromethoxy)phenyl]cyclopropanecarbonitrile:

As above. From 2-(4-trifluoromethoxyphenyl)acetonitrile (4.36g, 21.68mmol) to yield 1-[4-(trifluoromethoxy)phenyl]cyclopropanecarbonitrile (4.08g, 17.96mmol, 83%). ¹H NMR (400 MHz, CDCl₃) δ ppm 7.33 (d, 2H), 7.22 (d, 2H), 1.71 (m, 2H), 1.40 (m, 2H).

### 1-[2-(trifluoromethyl)phenyl]cyclopropanecarbonitrile:

As above: From 2-(2-trifluoromethylphenyl)acetonitrile (5.00g, 27.01mmol) to yield 1-[2-(trifluoromethyl)phenyl]cyclopropanecarbonitrile (5.58g, 26.42mmol, 98%). ¹H NMR (400 MHz, CDCl₃) δ ppm 7.72-7.48 (m, 4H), 1.77 (m, 2H), 1.44 (m, 2H).

### 1-[3-(trifluoromethyl)phenyl]cyclopropanecarbonitrile:

As above: From 2-(3-trifluoromethylphenyl)acetonitrile (5.00g, 27.01mmol) to yield 1-[3-(trifluoromethyl)phenyl]cyclopropanecarbonitrile (5.05g, 23.91mmol, 89%). ¹H NMR (400 MHz, CDCl₃) δ ppm 7.57-7.49 (m, 4H), 1.80 (m, 2H), 1.46 (m, 2H).

### 1-(4-morpholinophenyl)cyclopropanecarbonitrile:

To a mixture of 18ml of concentrated H₂SO₄ and 18ml of 65% HNO₃ solution, cooled on an ice bath and under a nitrogen atmosphere, was added neat 1-phenylcyclopropanecarbonitrile (10.00g, 69.84mmol). The reaction mixture was stirred for 15 minutes on ice, warmed to room temperature and stirred for an additional 30 minutes after which TLC (25% EtOAc in PE (40-60)) indicated complete consumption of the starting material. The reaction mixture was poured in water, the solids were filtered, washed with water, dried and recrystallized from EtOH to give 1-(4-nitrophenyl)cyclopropanecarbonitrile (9.47g, 50.32mmol, 72%). ¹H NMR (400 MHz, CDCl₃) δ ppm 8.21 (d, 2H), 7.43 (d, 2H), 1.91 (m, 2H), 1.54 (m, 2H). To a mixture of the above nitro compound (2.95g, 15.96mmol) in 50ml EtOH and 50ml water containing NH₄Cl (8.52g, 159.6mmol) at 50°C under a nitrogen atmosphere was added portionwise iron powder (4.45g, 79.65mmol). The resulting reaction mixture was stirred for 45 minutes after which TLC (25% EtOAc in PE (40-60)) indicated complete consumption of the starting material. The reaction mixture was cooled, filtered over celite and the filter cake was washed with EtOH. The filtrate was evaporated and the residue was partitioned between EtOAc and water. The aqueous layer was extracted with EtOAc and the combined organic layers were washed with brine, dried over MgSO₄ and evaporated to give 1-(4-aminophenyl)cyclopropanecarbonitrile (2.27g, 14.35mmol, 90%). ¹H NMR (400 MHz, CDCl₃) δ ppm 7.09 (d, 2H), 6.64 (d, 2H), 3.70 (bs, 2H), 1.60 (m, 2H), 1.28 (m, 2H). A mixture of the above amine (2.25g, 14.22mmol), 1-chloro-2-(2-chloroethoxy)ethane (2.44g, 17.07mmol), sodium iodide (5.33g, 35.56mmol) and K₂CO₃ (3.93g, 28.45mmol) in 25ml NMP was stirred for 3h at 120°C under a nitrogen atmosphere after which TLC (25% EtOAc in PE (40-60)) indicated complete consumption of the starting material. The residue was partitioned between EtOAc and water. The aqueous layer was extracted with EtOAc and the combined organic layers were washed with brine, dried over MgSO₄ and evaporated. The residue was purified by column chromatography (silica, 5% EtOAc in DCM) to give 1-(4-morpholinophenyl)-cyclopropanecarbonitrile (2.60g, 11.39mmol, 80%) as a crystalline solid. NMR (400 MHz, CDCl₃) δ ppm 7.21 (d, 2H), 6.87 (d, 2H), 3.85 (d, 4H), 3.15 (d, 4H), 1.64 (m, 2H), 1.30 (m, 2H).

### 1-(4-chlorophenyl]cyclopropanecarbaldehyde:

To a solution of 1-(4-chlorophenyl)cyclopropanecarbonitrile (2.48g, 13.96mmol) in 215mL of DCM, cooled on an ice bath under a nitrogen atmosphere, DIBAL-H (1M in DCM) (18mL, 18mmol) was added dropwise. After the reaction mixture was stirred for 16h, TLC (10% EtOAc in PE (40-60)) indicated complete consumption of the starting material. 1N HCI solution (40ml) was added dropwise to the reaction mixture at 0°C. Additional DCM was added to facilitate stirring. The layers were separated, and the organic fraction was washed twice with water. The combined aqueous layers were extracted with DCM and the combined organic layers were washed with brine, dried over MgSO₄ and evaporated. The residue was purified by flash column chromatography (silica, 15% EtOAc in PE (40/60)) to give 1-(4-chlorophenyl]cyclopropanecarbaldehyde (2.10g, 11.63mmol, 83%) as a yellow oil which crystallizes on standing. ¹H NMR (400 MHz, CDCl₃) δ ppm 9.16 (s, 1H), 7.33 (d, 2H), 7.23 (d, 2H), 1.58 (m, 2H), 1.38 (m, 2H).

### 1-(4-methoxyphenyl)cyclopropanecarbaldehyde:

As above. From 1-(4-methoxyphenyl)cyclopropanecarbonitrile (3.51g, 20.20mmol) to give 1-(4-methoxyphenyl)cyclopropanecarbaldehyde (2.69g, 15.27mmol, 76%) after purification by column chromatography (silica, 5% EtOAc in DCM). ¹H NMR (400 MHz, CDCl₃) δ ppm 9.23 (s, 1H), 7.23 (d, 2H), 6.90 (d, 2H), 3.81 (s, 3H), 1.54 (m, 2H), 1.36 (m, 2H).

### 1-(4-trifluoromethoxyphenyl)cyclopropanecarbaldehyde:

As above. From 1-(4-trifluoromethoxyphenyl)cyclopropanecarbonitrile (2.50g, 11.00mmol) to give 1-(4-methoxyphenyl)cyclopropanecarbaldehyde (2.05g, 8.91mmol, 81%) after purification by column chromatography (silica, 15% EtOAc in PE(40-60)). ¹H NMR (400 MHz, CDCl₃) δ ppm 9.23 (s, 1H), 7.23 (d, 2H), 6.90 (d, 2H), 3.81 (s, 3H), 1.54 (m, 2H), 1.36 (m, 2H).

### 1-(2-trifluorophenyl)cyclopropanecarbaldehyde:

As above. From 1-(2-trifluorophenyl)cyclopropanecarbonitrile (2.50g, 11.84mmol) to give 1-(2-trifluorophenyl)cyclopropanecarbaldehyde (1.83g, 8.54mmol, 72%) after purification by column chromatography (silica, 15% EtOAc in PE(40-60)). ¹H NMR (400 MHz, CDCl₃) δ ppm 9.13 (s, 1H), 7.73-7.23 (m, 4H), 1.70 (m, 2H), 1.49 (m, 2H).

### 1-(3-trifluorophenyl)cyclopropanecarbaldehyde:

As above. From 1-(3-trifluorophenyl)cyclopropanecarbonitrile (2.50g, 11.84mmol) to give 1-(3-trifluorophenyl)cyclopropanecarbaldehyde (2.27g, 10.60mmol, 90%) after purification by column chromatography (silica, 5% EtOAc in DCM). ¹H NMR (400 MHz, CDCl₃) δ ppm 9.15 (s, 1H), 7.58-7.47 (m, 4H), 1.63 (m, 2H), 1.45 (m, 2H).

### 1-(4-morpholinophenyl)cyclopropanecarbaldehyde:

As above. From 1-(4-morpholinophenyl)cyclopropanecarbonitrile (2.10g, 9.20mmol) to give 1-(4-morpholinophenyl)cyclopropanecarbaldehyde (1.69g, 7.31 mmol, 79%) after purification by column chromatography (silica, 10% EtOAc in DCM). ¹H NMR (400 MHz, CDCl₃) δ ppm 9.25 (s, 1H), 7.21 (d, 2H), 6.90 (d, 2H), 3.86 (d, 4H), 3.16 (d, 4H), 1.53 (m, 2H), 1.35 (m, 2H).

### 1-chloro-4-(1-ethynylcyclopropyl)benzene:

To a solution of 1-(4-chlorophenyl]cyclopropanecarbaldehyde (2.03g, 11.24mmol) in 8mL MeOH, cooled on an ice bath under a nitrogen atmosphere, a solution of dimethyl (1-diazo-2-oxopropyl)phosphonate (2.59g, 13.49mmol) in 5mL MeOH was added followed by the addition of K₂CO₃ (3.11g, 22.48mmol). After stirring 16h TLC (15% EtOAc in PE (40-60)) indicated complete consumption of the starting material. The reaction mixture was filtered over a celite cake and washed with MeOH. The filtrate was evaporated and diluted with EtOAc and washed with water, brine, dried over MgSO₄ and evaporated. The residue was purified by flash column chromatography (silica, PE (40-60)) to give 1-chloro-4-(1-ethynylcyclopropyl)benzene (576mg, 3.26mmol, 29%) as a colourless oil. ¹H NMR (400 MHz, CDCl₃) δ ppm 7.26 (s, 4H), 2.11 (s, 1H), 1.46 (m, 2H), 1.22 (m, 2H).

### 1-(1-ethynylcyclopropyl)-4-methoxybenzene:

As above. From 1-(4-methoxyphenyl)cyclopropanecarbaldehyde (2.69g, 15.30mmol) to give 1-(1-ethynylcyclopropyl)-4-methoxybenzene (2.51g, 14.57mmol, 95%). ¹H NMR (400 MHz, CDCl₃) δ ppm 7.27 (d, 2H), 6.84 (d, 2H), 3.79 (s, 3H), 2.06 (s, 1H), 1.39 (m, 2H), 1.17 (m, 2H).

### 1-(1-ethynylcyclopropyl)-4-(trifluoromethoxy)benzene:

As above. From 1-(4-trifluoromethoxyphenyl)cyclopropanecarbaldehyde (1.56g, 6.78mmol) to give 1-(1-ethynylcyclopropyl)-4-(trifluoromethoxy)benzene (1.48g, 6.54mmol, 97%). ¹H NMR (400 MHz, CDCl₃) δ ppm 7.35 (d, 2H), 7.14 (d, 2H), 2.12 (s, 1H), 1.48 (m, 2H), 1.23 (m,2H).

### 1-(1-ethynylcyclopropyl)-2-(trifluoromethyl)benzene:

As above. From 1-(2-trifluorophenyl)cyclopropanecarbaldehyde (1.83g, 8.54mmol) to give 1-(1-ethynylcyclopropyl)-2-(trifluoromethyl)benzene (764mg, 3.55mmol, 42%). ¹H NMR (400 MHz, CDCl₃) δ ppm 7.66-7.38 (m, 4H), 1.93 (s, 1H), 1.45 (m, 2H), 1.25 (m, 2H).

### 1-(1-ethynylcyclopropyl)-3-(trifluoromethyl)benzene:

As above. From 1-(3-trifluorophenyl)cyclopropanecarbaldehyde (2.27g, 10.60mmol) to give 1-(1-ethynylcyclopropyl)-3-(trifluoromethyl)benzene (1.77g, 8.42mmol, 79%). ¹H NMR (400 MHz, CDCl₃) δ ppm 7.58-7.40 (m, 4H), 1.99 (s, 1H), 1.46 (m, 2H), 1.25 (m, 2H).

### 4-[4-(1-ethynylcyclopropyl)phenyl]morpholine:

As above. From 1-(4-morpholinophenyl)cyclopropanecarbaldehyde (1.69g, 7.40mmol) to give 4-[4-(1-ethynylcyclopropyl)phenyl]morpholine (1.66g, 7.30mmol, 99%). ¹H NMR (400 MHz, CDCl₃) δ ppm 7.26 (d, 2H), 6.85 (d, 2H), 3.85 (d, 4H), 3.12 (d, 4H), 2.06 (s, 1H), 1.38 (m, 2H), 1.17 (m, 2H).

### [2-[1-(4-chlorophenyl)cyclopropyl]vinyl]boronic acid:

To a solution of 1-chloro-4-(1-ethynylcyclopropyl)benzene (576mg, 3.26mmol) in 6mL THF a solution of catecholborane (430mg, 3.59mmol) in 2mL THF was added and the resulting mixture was stirred overnight at 70°C under a nitrogen atmosphere. After cooling, water was added and the reaction mixture was stirred for 3h at room temperature. The resulting solids were filtered and dried to give [2-[1-(4-chlorophenyl)cyclopropyl]vinyl]boronic acid (585mg, 2.63mmol, 81%) which was used directly in the next step.

### [2-[1-(4-methoxyphenyl)cyclopropyl]vinyl]boronic acid:

As above: From 1-(1-ethynylcyclopropyl)-4-methoxybenzene (1.51g, 8.74mmol). No precipitation was observed after the hydrolysis is water. The slurry was extracted into EtOAc, washed with brine, dried over MgSO₄ and evaporated to give a mixture of the boronic acid and catechol which was used directly in the next step.

### [2-[1-(4-trifluoromethoxyphenyl)cyclopropyl]vinyl]boronic acid:

As above: From 1-(1-ethynylcyclopropyl)-4-trifluoromethoxybenzene (556mg, 2.46mmol). No precipitation was observed after the hydrolysis is water. The slurry was extracted into EtOAc, washed with brine, dried over MgSO₄ and evaporated to give a mixture of the boronic acid and catechol which was used directly in the next step.

### [2-[1-(2-trifluoromethylphenyl)cyclopropyl]vinyl]boronic acid:

As above: From 1-(1-ethynylcyclopropyl)-2-trifluoromethylbenzene (530mg, 2.52mmol). No precipitation was observed after the hydrolysis is water. The slurry was extracted into EtOAc, washed with brine, dried over MgSO₄ and evaporated to give a mixture of the boronic acid and catechol which was used directly in the next step.

### [2-[1-(3-trifluoromethylphenyl)cyclopropyl]vinyl]boronic acid:

As above: From 1-(1-ethynylcyclopropyl)-3-trifluoromethylbenzene (586mg, 2.88mmol). No precipitation was observed after the hydrolysis is water. The slurry was extracted into EtOAc, washed with brine, dried over MgSO₄ and evaporated to give a mixture of the boronic acid and catechol which was used directly in the next step.

### [2-[1-(4-morpholinophenyl)cyclopropyl]vinyl]boronic acid:

As above. From 4-[4-(1-ethynylcyclopropyl)phenyl]morpholine (571 mg, 2.51 mmol) to give [2-[1-(4-morpholinophenyl)cyclopropyl]vinyl]boronic acid (554mg, 2.03mmol, 81%) which was used directly in the next step.

### 5-[2-[1-(4-chlorophenyl)cyclopropyl]vinyl]-6-methylpyrimidine-2,4-diamine (GHR_0011):

[2-[1-(4-chlorophenyl)cyclopropyl]vinyl]boronic acid (585mg, 2.63mmol) was added to a solution of 5-iodo-6-methyl-pyrimidine-2,4-diamine (500mg, 2.00mmol) in 25mL of THF and the resulting reaction mixture is purged with nitrogen. A solution of 10ml 2N Na₂CO₃ solution was added, the air was evacuated under high vacuum and filled with a nitrogen atmosphere. This procedure was repeated twice, (PPh₃)₄Pd(0) (575mg, 0.50mmol) was added and the reaction mixture was stirred at 70°C for 16h under a nitrogen atmosphere. The reaction mixture was diluted with EtOAc and washed twice with water. The combined aqueous layers were extracted with EtOAc and the combined organic layers were washed with brine, dried over MgSO₄ and evaporated. The residue was purified by column chromatography (silica, 20% i-PrOH/EtOAc), the fractions containing the product were pooled, evaporated, dissolved in a minimum amount of warm i-PrOH and injected into water. The solids were filtered, washed with water and dried to give 5-[2-[1-(4-chlorophenyl)cyclopropyl]vinyl]-6-methylpyrimidine-2,4-diamine (340mg, 1.13mmol, 57%). ¹H NMR (400 MHz, CDCl₃) δ ppm 7.34 (s, 4H), 5.88-5.73 (m, 6H), 2.01 (s, 3H), 1.16 (m, 2H), 1.04 (m, 2H).

### 5-[2-[1-(4-methoxyphenyl)cyclopropyl]vinyl]-6-methylpyrimidine-2,4-diamine (GHR_0003):

As above. From the 4-methoxyboronic acid-catechol mixture (890mg), 5-iodo-6-methylpyrimidine-2,4-diamine (810mg, 3.27mmol) and (PPh₃)₄Pd(0) (473mg, 0.41mmol) to give 5-[2-[1-(4-methoxyphenyl)-cyclopropyl]vinyl]-6-methylpyrimidine-2,4-diamine (200mg, 0.68mmol). ¹H NMR (400 MHz, CDCl₃) δ ppm 7.29 (s, 4H), 6.88 (d, 1H), 5.62 (m, 1H), 4.68 (m, 4H), 3.81 (s, 3H), 2.13 (s, 3H), 1.20 (m, 2H), 1.14 (m, 2H).

### 6-methyl-5-[2-[1-[4-(trifluoromethoxy)phenyl]cyclopropyl]vinyl]pyrimidine-2,4-diamine (GHR_0016):

As above. From the 4-trifluoromethoxyboronic acid-catechol mixture (650mg), 5-iodo-6-methylpyrimidine-2,4-diamine (418mg, 1.67mmol) and (PPh₃)₄Pd(0) (552mg, 0.48mmol) to give 6-methyl-5-[2-[1-[4-(trifluoromethoxy)phenyl]cyclopropyl]vinyl]pyrimidine-2,4-diamine (77mg, 0.22mmol). ¹H NMR (400 MHz, CDCl₃) δ ppm 7.39-7.18 (m, 5H), 5.55 (dd, 1H), 4.66 (m, 4H), 2.13 (s, 3H), 1.18 (m, 2H), 1.08 (m, 2H).

### 6-methyl-5-[2-[1-[2-(trifluoromethyl)phenyl]cyclopropyl]vinyl]pyrimidine-2,4-diamine (GHR_0043):

As above. From the 2-trifluoromethylboronic acid-catechol mixture (708mg), 5-iodo-6-methylpyrimidine-2,4-diamine (380mg, 1.52mmol) and (PPh₃)₄Pd(0) (639mg, 0.55mmol) to give 6-methyl-5-[2-[1-[2-(trifluoromethyl)phenyl]cyclopropyl]vinyl]pyrimidine-2,4-diamine (206mg, 0.62mmol). ¹H NMR (400 MHz, CDCl₃) δ ppm 7.69-7.40 (s, 4H), 5.54 (d, 1H), 5.32 (d, 1H), 4.71 (m, 4H), 2.16 (s, 3H), 1.31 (m, 2H), 1.16 (m, 2H).

### 6-methyl-5-[2-[1-[3-(trifluoromethyl)phenyl]cyclopropyl]vinyl]pyrimidine-2,4-diamine (GHR_0013):

As above. From the 3-trifluoromethylboronic acid-catechol mixture (736mg), 5-iodo-6-methylpyrimidine-2,4-diamine (431mg, 1.72mmol) and (PPh₃)₄Pd(0) (650mg, 0.58mmol) to give 6-methyl-5-[2-[1-[3-(trifluoromethyl)phenyl]cyclopropyl]vinyl]pyrimidine-2,4-diamine (54mg, 0.17mmol). ¹H NMR (400 MHz, CDCl₃) □ ppm 7.62-7.36 (s, 4H), 5.71 (d, 1H), 5.62 (d, 1H), 4.61 (m, 4H), 2.12 (s, 3H), 1.21 (m, 2H), 1.11 (m, 2H).

### 6-methyl-5-[2-[1-(4-morpholinophenyl)cyclopropyl]vinyl]pyrimidine-2,4-diamine (GHR_0017):

As above. From [2-[1-(4-morpholinophenyl)cyclopropyl]vinyl]boronic acid (554mg, 2.03mmol), 5-iodo-6-methylpyrimidine-2,4-diamine (380mg, 1.52mmol) and (PPh₃)₄Pd(0) (468mg, 0.41 mmol) to give 6-methyl-5-[2-[1-(4-morpholinophenyl)cyclopropyl]vinyl]pyrimidine-2,4-diamine (167mg, 0.48mmol, 32%). ¹H NMR (400 MHz, CDCl₃) δ ppm 7.18 (d, 2H), 6.87 (d, 2H), 5.70 (m, 6H), 3.71 (bs, 4H), 3.05 (bs, 4H), 1.98 (s, 3H), 1.06 (m, 2H), 0.97 (m, 2H).

### General scheme for the synthesis of ethynylpyrimidine-2-4-diamines:

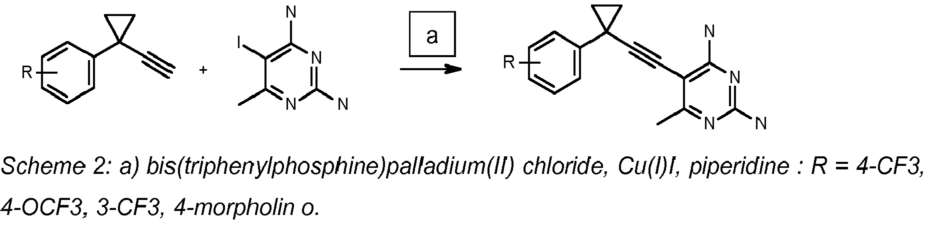

### Synthesis of 6-methyl-5-[2-[1-[4-(trifluoromethyl)phenyl]cyclopropyl]ethynyl]pyrimidine-2,4-diamine as a general example

### 6-methyl-5-[2-[1-[4-(trifluoromethyl)phenyl]cyclopropyl]ethynyl]pyrimidine-2,4-diamine (GHR_0012):

A mixture of 1-(1-ethynylcyclopropyl)-4-(trifluoromethyl)benzene (504mg, 2.40mmol), 5-iodo-6-methylpyrimidine-2,4-diamine (383mg, 1.53mmol), Cu(I)I (18mg, 0.10mmol) and piperidine (2.1ml, 26.86mmol) in 20ml DMF was degassed under high vacuum and purged with nitrogen. This procedure was repeated twice. Under a nitrogen atmosphere, bis(triphenylphosphine)palladium(II) chloride (93mg, 0.13mmol) was added and the resulting mixture was stirred over night at room temperature. The mixture was diluted with water, extracted twice with EtOAc and the combined organic layers were washed 3 times with 5% LiCI solution, dried over MgSO4, evaporated and purified by column chromatography (silica, 5% MeOH in DCM) to give 6-methyl-5-[2-[1-[4-(trifluoromethyl)phenyl]cyclopropyl]ethynyl]pyrimidine-2,4-diamine (440mg, 1.32mmol, 87%). ¹H NMR (400 MHz, CDCl₃) δ ppm 7.57 (d, 2H), 7.47 (d, 2H), 5.10 (bs, 2H), 4.81 (bs, 2H), 2.38 (s, 3H), 1.61 (m, 2H), 1.42 (m, 2H).

### 6-methyl-5-[2-[1-[4-(trifluoromethoxy)phenyl]cyclopropyl]ethynyl]pyrimidine-2,4-diamine (GHR_0039):

As above. From 1-(1-ethynylcyclopropyl)-4-(trifluoromethoxy)benzene (466mg, 2.06mmol), 5-iodo-6-methylpyrimidine-2,4-diamine (360mg, 1.44mmol), Cu(I)I (12mg, 0.06mmol), piperidine (2.75ml, 37.55mmol) and bis(triphenylphosphine)palladium(II) chloride (72mg, 0.10mmol) to give 6-methyl-5-[2-[1-[4-(trifluoromethoxy)phenyl]cyclopropyl]ethynyl]pyrimidine-2,4-diamine (211mg, 0.61mmol, 42%). ¹H NMR (400 MHz, CDCl₃) δ ppm 7.38 (d, 2H), 7.16 (d, 2H), 5.16 (bs, 2H), 4.92 (bs, 2H), 2.37 (s, 3H), 1.54 (m, 2H), 1.36 (m, 2H).

### 6-methyl-5-[2-[1-[3-(trifluoromethyl)phenyl]cyclopropyl]ethynyl]pyrimidine-2,4-diamine (GHR_0042):

As above. From 1-(1-ethynylcyclopropyl)-3-trifluoromethylbenzene (506mg, 2.41 mmol), 5-iodo-6-methylpyrimidine-2,4-diamine (421mg, 1.69mmol), Cu(I)I (14mg, 0.07mmol), piperidine (2.5ml, 34.14mmol) and bis(triphenylphosphine)palladium(II) chloride (85mg, 0.12mmol) to give 6-methyl-5-[2-[1-[3-(trifluoromethyl)phenyl]cyclopropyl]ethynyl]pyrimidine-2,4-diamine (377mg, 1.13mmol, 67%). ¹H NMR (400 MHz, DMSO-d₆) δ ppm 7.76 (s, 1H), 7.65 (m, 3H), 6.25 (bs, 2H), 6.15 (s, 2H), 2.21 (s, 3H), 1.65 (m, 2H), 1.44 (m, 2H).

### 6-methyl-5-[2-[1-(4-morpholinophenyl)cyclopropyl]ethynyl]pyrimidine-2,4-diamine (GHR_0041):

As above. From 4-[4-(1-ethynylcyclopropyl)phenyl]morpholine (423mg, 1.86mmol), 5-iodo-6-methylpyrimidine-2,4-diamine (326mg, 1.30mmol), Cu(I)I (11mg, 0.06mmol), piperidine (2.5ml, 34.14mmol) and bis(triphenylphosphine)palladium(II) chloride (65mg, 0.09mmol) to give 6-methyl-5-[2-[1-(4-morpholinophenyl)cyclopropyl]ethynyl]pyrimidine-2,4-diamine: (350mg, 1.00mmol, 77%). ¹H NMR (400 MHz, CDCl₃) δ ppm 7.28 (d, 2H), 6.86 (d, 2H), 5.10 (bs, 2H), 4.79 (bs, 2H), 3.85 (t, 4H), 3.13 (t, 4H), 2.36 (s, 3H), 1.30 (m, 2H), 1.21 (m, 2H).

### REFERENCES

Basu, R., Y. Qian, and J.J. Kopchick. 2018. MECHANISMS IN ENDOCRINOLOGY: Lessons from growth hormone receptor gene disrupted mice: Are there benefits of endocrine defects? Eur J Endocrinol.
Buchman, M., S. Bell, and J.J. Kopchick. 2018. Growth Hormone Discovery and Structure. Pediatr Endocrinol Rev. 16:2-10.
Chhabra, Y., Waters, M.J., Brooks, A.J. 2011. Role of the growth hormone-IGF-1 axis in cancer. Expert. Rev.Endocrinol. Metab.:71-84*.*
Christgen, M., and P. Derksen. 2015. Lobular breast cancer: molecular basis, mouse and cellular models. Breast Cancer Res. 17:16.
Dehkhoda, F., C.M.M. Lee, J. Medina, and A.J. Brooks. 2018. The Growth Hormone Receptor: Mechanism of Receptor Activation, Cell Signaling, and Physiological Aspects. Frontiers in endocrinology. 9:35.
Furqan, M., N. Mukhi, B. Lee, and D. Liu. 2013. Dysregulation of JAK-STAT pathway in hematological malignancies and JAK inhibitors for clinical application. Biomarker research. 1:5.
Guevara-Aguirre, J., P. Balasubramanian, M. Guevara-Aguirre, Wei, F. Madia, C.W. Cheng, D. Hwang, A. Martin-Montalvo, J. Saaved, S. Ingles, R. de Cabo, P. Cohen, and V.D. Longo. 2011. Growth hormone receptor deficiency is associated with a major reduction in pro-aging signaling, cancer, and diabetes in humans. Science translational medicine. 3:70
Hollestelle, A., J.K. Peeters, M. Smid, M. Timmermans, L.C. Verhoog, P.J. Westenend, A.A. Heine, A. Chan, A.M. Sieuwerts, E.A. Wiemer, J.G. Klijn, P.J. van der Spek, J.A. Foekens, M. Schutte, M.A. den Bakker, and J.W. Martens. 2013. Loss of E-cadherin is not a necessity for epithelial to mesenchymal transition in human breast cancer. Breast Cancer Res Treat. 138:47-57.
Jessiman, A.G., D.D. Matson, and F.D. Moore. 1959. Hypophysectomy in the treatment of breast cancer. N Engl J Med. 261:1199-1207.
Laron, Z. 2015. Lessons from 50 Years of Study of Laron Syndrome. Endocrine practice : official journal of the American College of Endocrinology and the American Association of Clinical Endocrinologists. 21:1395-1402.
Lu, M., J.U. Flanagan, R.J. Langley, M.P. Hay, and J.K. Perry. 2019. Targeting growth hormone function: strategies and therapeutic applications. Signal transduction and targeted therapy. 4:3
Malergue, F., A. van Agthoven, C. Scifo, D. Egan, and G.J. Strous. 2015. utomation of a phospho-STAT5 staining procedure for flow cytometry for application in drug discovery. J Biomol Screen. 20:416-421.
Minoia, M., E. Gentilin, D. Mole, M. Rossi, C. Filieri, F. Tagliati, A. Baroni, M.R. Ambrosio, E. degli Uberti, and M.C. Zatelli. 2012. Growth hormone receptor blockade inhibits growth hormone-induced chemoresistance by restoring cytotoxic-induced apoptosis in breast cancer cells independently of estrogen receptor expression. J Clin Endocrinol Metab. 97:E907-916.
Nashiro, K., J. Guevara-Aguirre, M.N. Braskie, G.W. Hafzalla, R. Velasco, P. Balasubramanian, M. Wei, P.M. Thompson, M. Mather M.D. Nelson, A. Guevara, E. Teran, and V.D. Longo. 2017. Brain Structure and Function Associated with Younger Adults in Growth Hormone Receptor-Deficient Humans. J Neurosci. 37:1696-1707.
Nespital, T., L.M. van der Velden, A. Mensinga, E.D. van der Vaart, and G.J. Strous. 2016. Fos-Zippered GH Receptor Cytosolic Tails Act as Jak2 Substrates and Signal Transducers. Mol Endocrinol. 30:290-301.
Ranke, M.B., and J.M. Wit. 2018. Growth hormone - past, present and future. Nat Rev Endocrinol. 14:285-300 http://www.ncbi.nlm.nih.gov/pubmed/29546874.
Sedek, M., L.M. van der Velden, and G.J. Strous. 2014. Multimeric growth hormone receptor complexes serve as signaling platforms. J. Biol. Chem. 289:65-73.
Theodoropoulou, M., and G.K. Stalla. 2013. Somatostatin receptors: from signaling to clinical practice. Frontiers in neuroendocrinology. 34:228-252.
Timmermans-Sprang, E.P.M., A. Gracanin, and J.A. Mol. 2017. Molecular Signaling of Progesterone, Growth Hormone, Wnt, and HE in Mammary Glands of Dogs, Rodents, and Humans: New Treatment Target Identification. Frontiers in veterinary science. 4:53.
Vermeulen, J.F., A.S. van Brussel, A. Adams, W.P. Mali, E. van der Wall, P.J. van Diest, and P.W. Derksen. 2013. Near-infrared fluorescence molecular imaging of ductal carcinoma in situ with CD44v6-specific antibodies in mice: a preclinical study. Molecular imaging and biology: MIB : the official publication of the Academy of Molecular Imaging. 15:290-298.
Zhou, Y., B.C. Xu, H.G. Maheshwari, L. He, M. Reed, M. Lozykowski, S. Okada, L. Cataldo, K. Coschigamo, T.E. Wagner, G. Baumann, and J.J. Kopchick. 1997. A mammalian model for Laron syndrome produced by targeted disruption of the mouse growth hormone receptor/binding protein gene (the Laron mouse). Proc Natl Acad Sci U S A. 94:13215-13220.

## Claims

1. A compound according to formula 1 for use in the treatment or prevention of a disease in a subject, 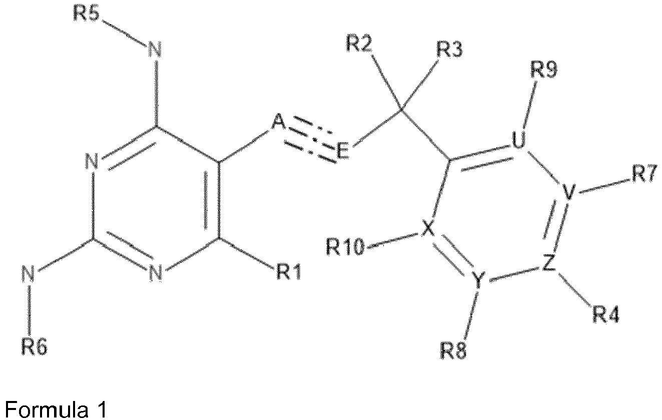 wherein
A and E represent carbon atoms with binding between A and E with a single bond or a double bond in E-configuration or a triple bond.
U, V, X, Y and Z represent carbon atoms or one of them represents an unsubstituted N to form a pyridine,
R¹ is (1C-3C)-alkyl or 3,3,3-trifluoropropyl,
R² is methyl and R³ is hydrogen or methyl, or R² and R³ form together with the carbon to which they are attached a cyclopropane, or R² and R³ form together with the carbon to which they are attached a cyclopentane,
R⁴, R⁷, R⁸, R⁹ and R¹⁰ are hydrogen, halogen, trifluoromethyl, methoxy, trifluoromethoxy, morpholine or methylsulfonyl,
R⁵ and R⁶ are hydrogen, ethyl or R⁵ or R⁶ are pyridin-2-ylmethyl, or a pharmaceutically acceptable salt thereof.

2. The compound for use according to claim 1 wherein the disease is a growth hormone receptor dependent disease.

3. The compound for use according to claim 1 or 2 wherein treatment or prevention is selected from the group consisting of the treatment or prevention of cancer, preventing or inhibiting cancer metastasis, treatment or prevention of diabetes, treatment of chronic inflammation such as rheumatoid arthritis and Crohn's disease, treatment of acromegaly, treatment or prevention of neurodegenerative diseases, treatment and prevention of idiopathic pulmonary disease and other occurrences of fibrosis, treatment and prevention of autoimmune diseases, such as Lupus erythematodes and macular degeneration, improving long- and short-term memory and increasing health span.

4. The compound for use according to any one of claims 1 - 3 wherein the disease is cancer.

5. The compound for use according to any one of claims 1 - 4 wherein R5 is pyridin-2-ylmethyl,

6. The compound for use according to any one of claims 1 - 5 wherein R5, R6, R7, R8 and R9 are hydrogen.

7. The compound for use according to claim 6 wherein
R1 is methyl,
R2 and R3 form together with the carbon to which they are attached a cyclopropane and
R4 is chloro or trifluoromethyl.

8. The compound for use according to any one of claims 1 - 7 wherein the compound is selected from the group consisting of 6-methyl-5-[2-{1-[4-(trifluoromethyl)phenyl]cyclopropyl}ethenyl]pyrimidine-2,4-diamine, 5-{[1-(4-chlorophenyl)cyclopropyl]ethynyl}-6-methylpyrimidine-2,4-diamine, 5-{[1-(4-chlorophenyl)cyclopropyl]ethynyl}-6-methylpyrimidine-2,4-diamine, 5-[3-(4-fluorophenyl)butyl]-6-methylpyrimidine-2,4-diamine, 5-{3-methyl-3-[4-(trifluoromethyl)phenyl]but-1-en-1-yl}-6-(3,3,3-trifluoropropyl)pyrimidine-2,4-diamine, 6-methyl-5-[3-methyl-3-[4-(trifluoromethyl)phenyl]but-1-en-1-yl]-N4-[(pyridin-2-yl)methyl]pyrimidine-2,4-diamine, 6-methyl-5-{3-methyl-3-[4-(trifluoromethyl)phenyl]but-1-en-1-yl}-N2-[(pyridin-2-yl)methyl]pyrimidine-2,4-diamine and 6-methyl-5-{3-[4-(trifluoromethyl)phenyl]but-1-en-1-yl}pyrimidine-2,4-diamine.

9. A composition comprising a compound according to formula 1 for use in the treatment or prevention of a disease in a subject, 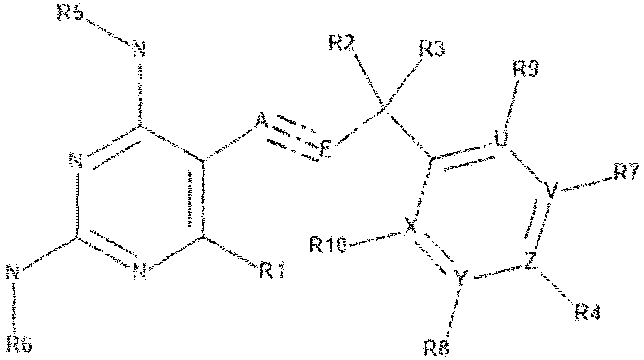 wherein
A and E represent carbon atoms with binding between A and E with a single bond or a double bond in E-configuration or a triple bond.
U, V, X, Y and Z represent carbon atoms or one of them represents an unsubstituted N to form a pyridine,
R¹ is (1C-3C)-alkyl or 3,3,3-trifluoropropyl,
R² is methyl and R³ is hydrogen or methyl, or R² and R³ form together with the carbon to which they are attached a cyclopropane, or R² and R³ form together with the carbon to which they are attached a cyclopentane,
R⁴, R⁷, R⁸, R⁹ and R¹⁰ are hydrogen, halogen, trifluoromethyl, methoxy, trifluoromethoxy, morpholine or methylsulfonyl and
R5 and R6 are hydrogen, ethyl or R5 or R6 are pyridin-2-ylmethyl

10. The composition for use according to claim 9 wherein the disease is a growth hormone receptor-dependent disease.

11. The composition for use according to claim 9 or 10 wherein treatment or prevention is selected from the group consisting of the treatment or prevention of cancer, preventing or inhibiting cancer metastasis, treatment or prevention of diabetes, treatment of chronic inflammation such as rheumatoid arthritis and Crohn's disease, treatment of acromegaly, treatment or prevention of neurodegenerative diseases, treatment and prevention of idiopathic pulmonary disease and other occurrences of fibrosis, treatment and prevention of autoimmune diseases, such as Lupus erythematosus and macular degeneration, improving long- and short-term memory and increasing health span.

12. The composition for use according to any one of claims 9 - 11 wherein the disease is cancer.

13. The composition according to claim 12 wherein R5 is pyridin-2-ylmethyl,

14. The composition for use according to claim 9 or 10 wherein R5, R6, R7, R8 and R9 are hydrogen.

15. The composition for use according to claim 14 wherein
R1 is methyl,
R2 and R3 form together with the carbon to which they are attached a cyclopropane and
R4 is chloro or trifluoromethyl.

16. The composition for use according to any one of claims 9 - 15, wherein the compound is selected from the group consisting of 6-methyl-5-[2-{1-[4-(trifluoromethyl)phenyl]cyclopropyl}ethenyl]pyrimidine-2,4-diamine, 5-{[1-(4-chlorophenyl)cyclopropyl]ethynyl}-6-methylpyrimidine-2,4-diamine, 5-{[1-(4-chlorophenyl)cyclopropyl]ethynyl}-6-methylpyrimidine-2,4-diamine, 5-[3-(4-fluorophenyl)butyl]-6-methylpyrimidine-2,4-diamine, 5-{3-methyl-3-[4-(trifluoromethyl)phenyl]but-1-en-1-yl}-6-(3,3,3-trifluoropropyl)pyrimidine-2,4-diamine, 6-methyl-5-[3-methyl-3-[4-(trifluoromethyl)phenyl]but-1-en-1-yl]- N4-[(pyridin-2-yl)methyl]pyrimidine-2,4-diamine, 6-methyl-5-{3-methyl-3-[4-(trifluoromethyl)phenyl]but-1-en-1-yl}-N2-[(pyridin-2-yl)methyl]pyrimidine-2,4-diamine and 6-methyl-5-{3-[4-(trifluoromethyl)phenyl]but-1-en-1-yl}pyrimidine-2,4-diamine.

17. The compound for use according to claim 4 or the composition for use according to claim 12 wherein the cancer is selected from the group consisting of melanoma, acute myeloid leukemia, chronic lymphocytic leukemia, colorectal cancer, renal cell carcinoma, breast cancer, lung cancer, ovarian cancer, fallopian tube carcinoma, primary peritoneal carcinoma, cervical cancer, gastric cancer, liver cancer, pancreatic cancer, thyroid cancer, glioma, non-Hodgkin's lymphoma, and Hodgkin's lymphoma.

18. A compound for use according to any one of claims 1 - 8 or 17, or a composition for use according to any one of claims 9 - 17 wherein the subject is selected from the group consisting of a human, an animal, a mammal, a primate, a higher primate, a sheep, a dog, a rodent, a mouse, a rat, a guinea pig, a goat, a pig, a cat, a rabbit, and a cow.
